# EUROPÄISCHE PATENTSCHRIFT

(11) **EP 2 355 754 B1**
(45) Veröffentlichungstag und Bekanntmachung des Hinweises auf die Patenterteilung: **18.04.2018**
(21) Anmeldenummer: 09778635.4
(22) Anmeldetag: 21.09.2009
(51) Int. Cl.: A61F 2/90

(54) **IMPLANTAT**
IMPLANT
IMPLANT

(30) Priorität: 23.09.2008 DE 102008048417
(43) Veröffentlichungstag der Anmeldung: 17.08.2011
(73) Patentinhaber: Acandis GmbH & Co. KG, 76327 Pfinztal (DE)
(72) Erfinder: CATTANEO, Giorgio, 76199 Karlsruhe (DE)
(74) Vertreter: Kilchert, Jochen
(86) Internationale Anmeldenummer: PCT/EP2009/006810
(87) Internationale Veröffentlichungsnummer: WO 2010/034453

(56) Entgegenhaltungen:
- EP-A- 1 068 876
- WO-A1-01/52771
- US-A- 4 610 688
- US-A1- 2002 165 597

## Beschreibung

Die Erfindung betrifft ein Implantat gemäß dem Oberbegriff des Patentanspruchs 1. Ein derartiges Implantat ist beispielsweise aus der US 6,258,115 B1 bekannt.

US 6,258,115 B1 offenbart einen Stent mit einer rotationssymmetrischen rohrförmigen Gitterstruktur, die aus einem Drahtgeflecht gebildet ist. Die Maschengröße des Drahtgeflechts ist bereichsweise unterschiedlich, so dass der Stent verschiedene Durchlässigkeiten aufweist. Vorzugsweise umfasst ein mittlerer Stentbereich größere Maschen, so dass der Stent beim Einsatz im Bereich einer Gefäßverzweigung einen ausreichenden Blutfluss in ein Nebengefäß ermöglicht.

Die Porosität bzw. Durchlässigkeit der Stentbereiche wird unter anderem durch den Flechtwinkel bestimmt. Der Flechtwinkel ist als derjenige Winkel definiert, der zwischen einem spiralförmig um die Rotationsachse des Stents gewundenen Draht in Draufsicht und einer zur Rotationsachse parallelen Geraden bzw. einer Projektion der Rotationsachse in die Umfangsebene gebildet ist. Beim Vergleich der Flechtwinkel zweier Drähte des Stents gemäß US 6,258,115 B1, wobei die zu vergleichenden Winkel in derselben Querschnittsebene des Stents betrachtet werden, ist festzustellen, dass die beiden Flechtwinkel gleich sind. Eine Variation des Flechtwinkels zur Änderung der Durchlässigkeitseigenschaften des Stents erfolgt nur in axialer Richtung des Stents. Dies ist insbesondere bei Betrachtung der Kreuzungs- bzw. Knotenpunkte ersichtlich, an denen sich zwei spiralförmig um die Rotationsachse verlaufende Drähte kreuzen. Die beiden spiralförmig gewundenen Drähte bzw. Coils weisen bezüglich einer zur Rotationsachse parallelen Geraden, die durch den Kreuzungsbereich verläuft, denselben Winkel (Flechtwinkel) auf.

Geflochtene Stents, wie beispielsweise der aus US 6,258,115 B1 bekannte Stent haben gegenüber Stents, die aus einem Vollmaterial geschnitten werden, beispielsweise gelaserten Stents den Vorteil, dass die Gitterstruktur durch eine geeignete Wahl der Drähte fein eingestellt werden kann. Die Strömungsverhältnisse, z.B. in Aneurysmen können durch die Feinmaschigkeit erheblich beeinflusst werden. Die feine Gitterstruktur senkt ferner das Risiko beim Platzieren eines Stents z.B. auf Plaque, dass Plaquepartikel sich ablösen und in die Blutbahn gelangen. Überdies wird das Zuwachsen des Gefäßes durch die feinen Maschen des geflochtenen Stents hindurch erschwert. Ein weiterer Vorteil geflochtener Stents ist deren Flexibilität. Die geflochtenen Drähte können aufeinander gleiten, so dass sich die Geometrie des Geflechtes, insbesondere der Winkel zwischen den einzelnen Drähten, in einem relativ weiten Bereich änderbar ist. Die gute Flexibilität geflochtener Stents macht diese für den Einsatz in Blutgefäßen mit kleinen Radien besonders geeignet. Die Feinmaschigkeit und gute Flexibilität geflochtener Stents wird durch die Verwendung dünner Drähte begünstigt.

Die Verwendung dünner Drähte schränkt jedoch die vom Stent auf die Gefäßwand wirkende Kraft (Radialkraft) ein. Die Radialkraft wird wie folgt erzeugt. Bei der Komprimierung des Stents im Zufuhrsystem (Katheter) wird dieser verformt, wobei die in Form von potentieller elastischer Energie gespeicherte Verformungskraft maximal ist. Wenn der Stent aus dem Katheter ins Gefäß entlassen wird, weitet sich dieser auf und verliert dabei einen Teil seiner potentiellen Energie. Da der Stent gegenüber dem Gefäß überdimensioniert ist, kann sich dieser nicht bis zu seinem ursprünglichen Durchmesser (vor dem Crimpen) aufweiten. Somit bleibt eine Restverformung gegenüber dem eingestellten Durchmesser des Stents bestehen. Die verbleibende Kraft, mit der der Stent versucht, zum ursprünglichen Durchmesser zurückzukehren, entspricht der auf die Gefäßwand wirkenden Radialkraft des Gittergeflechts.

Konkret kommt es bei der Verformung des geflochtenen Stents, d.h. bei der Durchmesseränderung zu einer Änderung der Winkel zwischen den Drähten, wobei zwei überkreuzende Drähte jeweils aufeinander gleiten. Im Kreuzungsbereich, d.h. im Bereich des Knotens wirkt die Reibung zwischen den beiden Drähten als Widerstand gegen die Winkeländerung. Überdies kommt es zu einer Verformung der einzelnen Drähte wie bei einer Feder, wenn das Geflecht komprimiert wird, wobei die Drähte tordiert, d.h. um ihre Achse verdreht werden. Wenn die radiale Komprimierung nachlässt, bspw. wenn der Stent aus dem Katheter entlassen wird, pressen die Drähte gegen die Gefäßwand, wobei eine Restverformung der Drähte bestehen bleibt. Die sich daraus ergebende Radialkraft auf die Gefäßwand ist eine Funktion des Momentes, mit dem die Drähte in den ursprünglichen Zustand drängen. Das Rückstellmoment bzw. die vom Stent bei der Verformung gespeicherte potentielle Energie ist bei dünnen Drähten relativ gering.

In der älteren, nachveröffentlichten DE 10 2008 036 428, die ebenfalls auf die Anmelderin zurückgeht, ist das Grundkonzept beschrieben, mit dem durch eine asymmetrische Flechtanordnung die Radialkraft bei rotationssymmetrischen medizinischen Vorrichtungen beeinflusst werden kann. Die vorliegende Erfindung bildet eine Weiterentwicklung dieses Grundlagenkonzepts. Die Weiterentwicklung ist besonders geeignet, aber nicht eingeschränkt auf den Einsatz bei Stents.

Der Erfindung liegt die Aufgabe zugrunde, ein Implantat, insbesondere einen Stent mit einem Gittergeflecht bzw. Drahtgeflecht so zu verbessern, dass eine im implantierten Zustand hohe Radialkraft auf die umgebende Gefäßwand erzielbar ist. Erfindungsgemäß wird diese Aufgabe durch den Gegenstand des Patentanspruchs 1 gelöst.

Die Erfindung beruht auf dem Gedanken, ein Implantat, aufweisend eine rotationssymmetrische Gitterstruktur mit wenigstens zwei spiralförmig um eine gemeinsame Rotationsachse R gewundenen Drahtelementen anzugeben, die mit einer gemeinsamen, senkrecht zur Rotationsachse R angeordneten Ebene L jeweils einen ersten und zweiten Schnittpunkt S', S" bilden, wobei durch den ersten Schnittpunkt S' eine erste Gerade R' und durch den zweiten Schnittpunkt S" eine zweite Gerade R" verlaufen, die jeweils parallel zur Rotationsachse R angeordnet sind und mit jeweils einem der beiden Drahtelemente einen spitzen Winkel einschließen. Dabei sind die beiden Winkel und die elastischen Eigenschaften der beiden Drahtelemente unterschiedlich.

Die Konfiguration der Flechtwinkel, d.h. der Winkel α' und α", bezieht sich auf den Ruhezustand der Vorrichtung. Die Bestimmung des Flechtwinkels wird bei der Vorrichtung im entspannten Zustand, d.h. ohne Einwirkung äußerer Kräfte, vorgenommen. Vorzugsweise ist die Vorrichtung zur Bestimmung der Winkel in axialer Richtung geradlinig ausgerichtet.

Die Erfindung unterscheidet sich von der älteren nachveröffentlichten DE 10 2008 036 428 dadurch, dass nicht nur die Flechtwinkel der beiden Drahtelemente unterschiedlich sind, sondern auch deren elastische Eigenschaften, also die drahtinhärenten Eigenschaften.

Die Erfindung hat den Vorteil, dass durch die hohe Radialkraft eine gute Fixierung der Vorrichtung, insbesondere des Stents in einem Körpergefäß erreicht wird. Dabei können dünne Drähte zum Einsatz kommen, so dass ebenfalls eine gute Flexibilität und Feinmaschigkeit des Stents erreicht wird. Die Erfindung bietet die Möglichkeit, eine medizinische Vorrichtung, insbesondere einen Stent zu schaffen, der im implantierten Zustand eine hohe Radialkraft und somit gute Fixierung im Gefäß mit den guten Flexibilitätseigenschaften und der Feinmaschigkeit eines geflochtenen Stents vereint.

Die Erfindung umfasst Flechtkonfiguration bzw. Ausrichtung der Drahtelemente, wobei der Winkel zwischen einem ersten Drahtelement und einer senkrechten Projektion der Rotationsachse R auf die Umfangsebene der Gitterstruktur gegenüber dem Winkel zwischen einem zweiten Drahtelement und einer weiteren senkrechten Projektion der Rotationsachse R auf die Umfangsfläche der Gitterstruktur und die elastischen Eigenschaften der Drahtelemente unterschiedlich sind. Der Vergleich der Winkel erfolgt auf derselben axialen Höhe der Gitterstruktur, d.h. in einer senkrecht zur Rotationsachse R orientierten Querschnittsebene L der Gitterstruktur. Auf diese Weise wird eine bezüglich der Winkel bzw. Flechtwinkel asymmetrische Gitterstruktur bereitgestellt.

Bei einer Durchmesser- oder Längenänderung verhalten sich separate Drahtspiralen bzw. Drahtelemente, die verschiedene Winkel aufweisen, unterschiedlich. Durch die asymmetrische Gitterstruktur bzw. die unterschiedlichen Winkel der Drahtelemente wird erreicht, dass bei der Komprimierung der geflochtenen, rohrförmigen Gitterstruktur wenigstens ein Drahtelement, das gegenüber dem anderen Drahtelement einen kleineren Flechtwinkel aufweist, in axialer Richtung bezüglich der Achse des Drahtelements gestreckt wird. Durch den Winkelunterschied zwischen den Drahtelementen verlängert sich das Drahtelement mit dem flacheren Winkel. Das Drahtelement mit dem größeren Flechtwinkel wird hingegen komprimiert bzw. gestaucht.

Zusätzlich zu den Drahtelementen mit unterschiedlichen Flechtwinkeln (asymmetrisches Geflecht) ist erfindungsgemäß vorgesehen, dass wenigstens zwei Drahtelemente mit unterschiedlichen Flechtwinkeln unterschiedliche elastische Eigenschaften aufweisen. Dabei handelt es sich um drahtinhärente Eigenschaften. Die unterschiedlichen elastischen Eigenschaften der Drahtelemente mit unterschiedlichen Flechtwinkeln, wobei nicht alle Drahtelemente mit unterschiedlichen Flechtwinkeln zwangsläufig unterschiedliche elastische Eigenschaften aufweisen müssen, schafft die Möglichkeit, das Kräftegleichgewicht zwischen den Drahtelementen mit den verschiedenen Flechtwinkeln einzustellen.

Der Winkelunterschied zwischen den Flechtwinkeln führt dazu, dass sich das Drahtgeflecht insgesamt verspannt, wobei die Drahtelemente mit kleinerem bzw. flacherem Flechtwinkel gedehnt und die Drahtelemente mit größerem Flechtwinkel komprimiert bzw. gestaucht werden. Es versteht sich, dass die Dehnung bzw. Komprimierung der Drahtelemente in Drahtlängsrichtung erfolgt. Durch die unterschiedlichen elastischen Eigenschaften der Drahtelemente mit verschiedenen Flechtwinkeln kann gezielt eingestellt werden, wie stark die prozentuale Dehnung bzw. prozentuale Komprimierung (Verkürzung) der Drahtelemente ist. Konkret kann eine übermäßige Komprimierung der Drahtelemente, die zu einer unerwünschten Verformung führen würde, beschränkt werden. Im Hinblick auf die aus dem Stand der Technik vorbekannten Stents, beispielsweise den Stent gemäß US 6,258,115 B1 hat die erfindungsgemäße Vorrichtung den Vorteil, dass eine hohe Radialkraft im implantierten Zustand erzeugbar ist, wobei die innere Verspannung des Drahtgeflechts aufgrund der verschiedenen elastischen Eigenschaften der jeweiligen Drahtelemente einstellbar ist. Im Hinblick auf ein Drahtgeflecht mit asymmetrischen Flechtwinkeln kann durch die Einstellung unterschiedlicher elastischer Eigenschaften der jeweiligen Drahtelemente mit verschiedenen Winkeln beispielsweise erreicht werden, dass ein entsprechend hergestellter Stent mit einer relativ niedrigen Kompressionskraft gecrimpt werden kann.

Die hohe Radialkraft wird bei dem erfindungsgemäßen Stent bzw. der erfindungsgemäßen Vorrichtung dadurch erreicht, dass die im Geflecht gespeicherte potentielle elastische Energie nicht nur in Form von Torsionsenergie, sondern in Form elastischer Verformungsenergie aufgrund der Streckung der Drahtelemente vorliegt.

Vorzugsweise umfassen die beiden Drahtelemente wenigstens ein erstes Drahtelement und wenigstens ein zweites Drahtelement. Das erste Drahtelement schließt vorzugsweise mit der ersten Geraden einen kleineren Winkel ein als das zweite Drahtelement mit der zweiten Geraden. Im Allgemeinen wird die vorzugsweise geflochtene, rohrförmige Gitterstruktur durch wenigstens zwei Drahtelemente gebildet, die spiralförmig gewickelt sind bzw. Drahtspiralen bilden. Die Länge des Drahtelements, das mit einem steileren bzw. größeren Winkel bezüglich der zur Rotationsachse parallelen Geraden, d.h. unter dem größeren Flechtwinkel gewunden ist, ist größer als die Länge des Drahtelements mit dem kleineren Flechtwinkel. Die durch das Drahtelement mit dem größeren Flechtwinkel gebildete Drahtspirale weist eine kleinere Steigung auf als die durch das Drahtelement mit dem kleineren Flechtwinkel gebildete Drahtspirale.

Bei einer Reduktion des Querschnittsdurchmessers des Geflechts, d.h. bei der Komprimierung des Geflechts, verlängern sich die Drahtspiralen in axialer Richtung, wobei das zweite Drahtelement mit dem größeren Flechtwinkel sich um einen höheren Wert verlängert, als das erste Drahtelement mit dem kleineren Winkel. Generell beeinflusst die Größe des Flechtwinkels auch den Grad der Verlängerung der jeweiligen Drahtspirale.

Bei einer bevorzugten Ausführungsform der erfindungsgemäßen medizinischen Vorrichtung ist das erste Drahtelement dehnbarer bzw. weist einen größeren Dehnungsbereich auf als das zweite Drahtelement, so dass das erste Drahtelement bei der Komprimierung der Gitterstruktur gestreckt, insbesondere elastisch gedehnt, wird. Unter Dehnung wird eine längsaxiale elastische Verformung des Drahtelementes verstanden, wobei geringfügige plastische Verformungen unschädlich sind. Aufgrund des größeren Dehnungsbereiches des ersten Drahtelementes mit dem kleineren Flechtwinkel kann die Vorrichtung mit einer geringeren äußeren Kraft komprimiert bzw. gecrimpt werden als bei einer Vorrichtung, deren Drahtelemente alle dieselbe Dehnbarkeit aufweisen. Überdies können die Drähte mit flachem Flechtwinkel aufgrund des größeren Dehnungsbereiches als die Drähte mit größerem Flechtwinkel für die Aufnahme in kleine Zufuhrsysteme sehr stark komprimiert werden. Das Risiko, dass die Drahtelemente aufgrund der starken Geflechtverlängerung brechen, ist aufgrund der elastischen Dehnung bzw. des großen Dehnungsbereiches der Drahtelemente mit kleinem Flechtwinkel gesenkt.

Die Einstellung der elastischen Eigenschaften beeinflusst überdies das Kräftegleichgewicht im Drahtgeflecht. Bei der Komprimierung der Gitterstruktur auf einen kleineren Querschnittsdurchmesser wirken auf die Drahtelemente unterschiedliche axiale Kräfte. Die Drahtelemente mit dem kleineren Winkel werden gestreckt, da die Drahtelemente mit dem kleineren Winkel im Wesentlichen kürzer sind als die Drahtelemente mit dem größeren Winkel. Bei der Komprimierung wirken Zugkräfte auf die ersten Drahtelemente. Aufgrund des Kräftegleichgewichts wirken auf die zweiten Drahtelemente mit dem größeren Winkel axiale Druckkräfte, die in Abhängigkeit der Materialeigenschaften der zweiten Drahtelemente zu einer Verkürzung der zweiten Drahtelemente führen können. Da die ersten Drahtelemente mit dem flacheren Flechtwinkel dehnbarer sind als die zweiten Drahtelemente, ist der Grad der Verlängerung der ersten Drahtelemente größer als der Grad der Verkürzung der zweiten Drahtelemente. Die Verformung bzw. Stauchung der zweiten Drahtelemente aufgrund von Druckkräften wird vermindert oder vermieden. Diese Wirkung kann dadurch unterstützt werden, dass eine größere Anzahl von zweiten Drahtelementen als ersten Drahtelementen vorgesehen ist, so dass die von einem ersten Drahtelement in Folge des Kräftegleichgewichts aufgebrachten Druckkräfte auf mehrere zweite Drahtelemente verteilt werden.

Vorzugsweise weist das erste Drahtelement ein kleineres Verhältnis zwischen Zugkraft und Dehnung auf als das zweite Drahtelement, so dass die Gitterstruktur mit einer für medizinische Zwecke angemessenen Kraft komprimierbar ist. Außerdem wird verhindert, dass die zweiten Drahtelemente mit dem größeren Flechtwinkel verformt oder beschädigt werden. Das Verhältnis zwischen Zugkraft und Dehnung gibt den Grad der Elastizität bzw. Dehnbarkeit des Drahtelements an.

Die unterschiedlichen elastischen Eigenschaften der Drahtelemente können unterschiedliche Werkstoffeigenschaften und/oder unterschiedliche Dimensionen, insbesondere Querschnittsdimensionen, wie Drahtdurchmesser bzw. Dicke und/oder unterschiedliche Geometrien der Drahtelemente umfassen.

Vorzugsweise weist das erste Drahtelement einen kleineren Elastizitätsmodul auf als das zweite Drahtelement. Die Elastizitätseigenschaften des ersten Drahtelements werden somit anhand des Werkstoffs derart beeinflusst, dass bei Komprimierung der Gitterstruktur das erste Drahtelement in axialer Richtung bezüglich der Achse des Drahtelements dehnbarer ist als das zweite Drahtelement.

In diesem Zusammenhang wird darauf hingewiesen, dass auch das zweite Drahtelement eine gewisse Dehnbarkeit bzw. Elastizität aufweisen kann. Aufgrund des Kräftegleichgewichts erfolgt bei der Komprimierung der Gitterstruktur im Wesentlichen eine Verkürzung bzw. Stauchung des zweiten Drahtelements.

Das Verhältnis zwischen dem Elastizitätsmodul des ersten Drahtelements und dem Elastizitätsmodul des zweiten Drahtelements kann höchstens 1:10, insbesondere 1:100, insbesondere höchstens 1:1000, betragen. Auf diese Weise kann die Kompressionskraft, die zur Überführung der Gitterstruktur vom expandierten in den komprimierten Zustand notwendig ist, durch die Elastizität der ersten Drahtelemente mit dem kleineren Winkel auf physiologisch geeignete Werte eingestellt werden. Gleichzeitig bewirkt die asymmetrische Anordnung der Drahtelemente eine Erhöhung der Radialkraft der Gitterstruktur. Die hohe Radialkraft, die mit der Erfindung erreicht wird, resultiert aus der Kombination des ersten Drahtelements mit einem flachen Flechtwinkel mit dem zweiten Drahtelement mit einem steilen Flechtwinkel. Durch die unterschiedlichen Winkel wird bei der Komprimierung der Gitterstruktur eine mechanische Spannung zwischen den Drahtelementen aufgebaut, die zu der erfindungsgemäßen Erhöhung der Radialkraft führt. Durch die elastischen Eigenschaften, insbesondere die unterschiedlichen Elastizitäten der Drahtelemente wird erreicht, dass die Gitterstruktur auf einen kleineren Querschnittsdurchmesser komprimierbar ist, wobei die dazu notwendige Kompressionskraft für den medizinischen Einsatz angemessen ist.

Für medizinische Anwendungen hat sich als zweckmäßig erwiesen, dass bei einer bevorzugten Ausführungsform der medizinischen Vorrichtung der Elastizitätsmodul des ersten Drahtelements zwischen 100 MPa und 5000 MPa, insbesondere zwischen 200 MPa und 4000 MPa, insbesondere zwischen 300 MPa und 3000 MPa, insbesondere zwischen 500 MPa und 2000 MPa, insbesondere zwischen 800 MPa und 1500 MPa, insbesondere zwischen 900 MPa und 1200 MPa, beträgt.

Vorzugsweise weist das erste Drahtelement eine Bruchdehnung von mehr als 10 %, 30 %, 50 %, 100 %, 150 %, 200 % auf. Die maximale Bruchdehnung bezeichnet den Verformungswert, bei dem das Drahtelement bricht oder reißt.

Alternativ oder zusätzlich können die unterschiedlichen elastischen Eigenschaften der Drahtelemente dadurch erreicht werden, dass das erste Drahtelement zumindest abschnittsweise eine kleinere Querschnittsfläche, insbesondere einen kleineren Querschnittsdurchmesser, aufweist, als das zweite Drahtelement.

Eine weitere Alternative oder zusätzliche Möglichkeit zur Variation der elastischen Eigenschaften besteht darin, dass die beiden Drahtelemente unterschiedliche Querschnittsprofile aufweisen. Das erste Drahtelement kann ein flaches, insbesondere rechteckiges oder ovales Querschnittsprofil aufweisen derart, dass das erste Drahtelement im Wesentlichen bandförmig ausgebildet ist. Beispielsweise können die zweiten Drahtelemente zylinderförmig ausgebildet sein, d.h. ein rundes Querschnittsprofil aufweisen, und die ersten Drahtelemente bandartig geformt sein. Es ist möglich, dass die beiden Drahtelemente dasselbe Material aufweisen und die elastischen Eigenschaften durch die unterschiedlichen Querschnittsprofile variiert werden. Auf diese Weise können die Drahtelemente einfach und sicher miteinander verbunden werden. Des Weiteren ist auf diese Weise die Biokompatibilität der medizinischen Vorrichtung gewährleistet.

Bei einer weiteren bevorzugten Ausführungsform der medizinischen Vorrichtung umfasst das erste Drahtelement wenigstens zwei Filamente, insbesondere ein Bündel aus mehreren Filamenten. Durch die Mehrzahl von Filamenten wird erreicht, dass das erste Drahtelement eine relativ hohe Zugfestigkeit aufweist, so dass eine ausreichend hohe Radialkraft zur Positionierung der Gitterstruktur in einem Körpergefäß gewährleistet ist.

Die beiden Drahtelemente können in derselben Richtung um die gemeinsame Rotationsachse gewunden, insbesondere geflochten, sein. In diesem Fall sind die beiden Flechtwinkel jeweils auf derselben Seite einer durch einen gemeinsamen Kreuzungsbereich der Drahtelemente verlaufenden Geraden angeordnet, so dass sich die Flechtwinkel überlappen. Die Differenz der beiden Flechtwinkel entspricht dem Differenzwinkel der beiden Drahtelemente, d.h. dem Winkel, der im Kreuzungsbereich zwischen den beiden Drahtelementen gebildet ist.

Alternativ können die beiden Drahtelemente in unterschiedlicher Richtung um die gemeinsame Rotationsachse gewunden, insbesondere geflochten, sein. Dabei sind die Flechtwinkel jeweils auf gegenüberliegenden Seiten einer durch einen gemeinsamen Kreuzungsbereich der Drahtelemente verlaufenden Geraden angeordnet, so dass sich die beiden Flechtwinkel in diesem Bereich zum Differenzwinkel zwischen den Drahtelementen ergänzen.

Vorzugsweise ist dem ersten Drahtelement und/oder dem zweiten Drahtelement wenigstens ein weiteres Drahtelement zugeordnet, das spiralförmig um die gemeinsame Rotationsachse in gegenläufiger Richtung zum ersten Drahtelement und/oder zum zweiten Drahtelement gewunden ist und denselben Winkel mit der ersten Geraden oder der zweiten Geraden einschließt wie das erste Drahtelement oder das zweite Drahtelement. Auf diese Weise wird eine vorteilhafte Kombination einer symmetrischen Geflechtkonfiguration mit einer asymmetrischen Geflechtkonfiguration erreicht. Insbesondere können zwei Drahtelemente, d.h. beispielsweise das erste Drahtelement und ein weiteres Drahtelement, unter demselben Flechtwinkel gegenläufig geflochten sein derart, dass sich die beiden Drahtelemente regelmäßig entlang der Gitterstruktur kreuzen. Ein zusätzliches Drahtelement, beispielsweise das zweite Drahtelement, kann unter einem anderen Flechtwinkel spiralförmig um die gemeinsame Rotationsachse gewunden sein, so dass das zweite Drahtelement die beiden symmetrisch geflochtenen Drahtelemente unregelmäßig entlang der Gitterstruktur kreuzt.

Bei einer bevorzugten Ausführungsform sind dem ersten Drahtelement und dem zweiten Drahtelement jeweils ein weiteres Drahtelement zugeordnet, wobei die weiteren Drahtelemente jeweils gegenläufig zum ersten oder zweiten Drahtelement gewunden sind und jeweils einen zum ersten oder zweiten Winkel α', α" symmetrischen Winkel aufweisen. Die Symmetrie der Winkel bezieht sich auf die die jeweilige Symmetrieachse bildende Gerade R', R". Die asymmetrische Gitterstruktur wird dabei durch zwei symmetrische Gittergeflechte gebildet, wobei das erste symmetrische Gittergeflecht ein erstes Drahtelement und ein weiteres Drahtelement, und das zweite symmetrische Gittergeflecht das zweite Drahtelement und ein weiteres Drahtelement umfasst. Die symmetrischen Gittergeflechte weisen unterschiedliche Flechtwinkel auf, so dass sich bei der Überlagerung bzw. Kombination bzw. beim Zusammenflechten der beiden Gittergeflechte insgesamt ein asymmetrisches Gittergeflecht ergibt. Die asymmetrische Gitterstruktur bzw. Flechtkonfiguration ist also durch paarweise in beide Drehrichtungen gewundene bzw. gewickelte Drahtelemente gebildet. Durch die besondere Kombination der symmetrischen Geflechtstrukturen wird die Stabilität der Gitterstruktur, insbesondere die Torsionsstabilität, erhöht. Insbesondere wird bei der Komprimierung der Gitterstruktur eine übermäßige Torsion der einzelnen Drahtelemente vermieden.

Vorzugsweise beträgt der Winkelunterschied zwischen dem ersten Winkel und dem zweiten Winkel wenigstens 2°, insbesondere wenigstens 5°, insbesondere wenigstens 8°, insbesondere wenigstens 10°, insbesondere wenigstens 20°, insbesondere wenigstens 30°, insbesondere wenigstens 40°, insbesondere wenigstens 45°, insbesondere wenigstens 50°, insbesondere wenigstens 60°, insbesondere wenigstens 70°. Die Radialkraft der medizinischen Vorrichtung wird vor allem durch die Größe des Winkelunterschieds zwischen dem ersten und dem zweiten Winkel bzw. durch den Grad der Asymmetrie bestimmt. Je größer der Winkelunterschied ist, desto höher ist die Radialkraft.

Ferner kann der Winkelunterschied zwischen dem ersten Winkel und dem zweiten Winkel höchstens 50°, insbesondere höchstens 45°, insbesondere höchstens 40°, insbesondere höchstens 30°, insbesondere höchstens 20°, insbesondere höchstens 10°, insbesondere höchstens 8°, insbesondere höchstens 6°, insbesondere höchstens 4°, insbesondere höchstens 2°, betragen. Die für eine Formänderung bzw. Längendehnung des ersten Drahtelements erforderliche Kraft wird mit abnehmendem Winkelunterschied kleiner bzw. mit zunehmendem Winkelunterschied größer. Durch eine geeignete Wahl der Werkstoff- oder Geometrieeigenschaften, insbesondere des Elastizitätsmoduls, der Drahtelemente kann im Zusammenhang mit dem Winkelunterschied gezielt eine für medizinische Zwecke geeignete radiale Rückstellkraft bei angemessener Kompressionskraft (Crimpen) eingestellt werden. Je höher die Dehnbarkeit der Drahtelemente ist, die unter Anderem durch einen kleineren Elastizitätsmodul oder einen kleineren Querschnittsdurchmesser einstellbar ist, umso kleiner ist die Rückstellkraft des Gittergeflechts. Die Obergrenze des Winkelunterschieds von 10°, insbesondere von 8°, insbesondere von 6°, insbesondere von 4°, insbesondere von 2°, ist für medizinische Zwecke besonders geeignet. Kleinere Winkelunterschiede als 2° sind möglich.

Der erste Winkel kann kleiner als 50°, insbesondere kleiner als 45°, insbesondere kleiner als 40°, insbesondere kleiner als 20° sein. Der zweite Winkel kann größer als 45°, insbesondere größer als 50°, insbesondere größer als 55°, insbesondere größer als 60°, insbesondere größer als 65°, insbesondere größer als 70°, sein.

Des Weiteren kann das Verhältnis der Anzahl der ersten Drahtelemente, d.h. der Drahtelemente mit dem ersten Winkel (kleiner Flechtwinkel) und der Anzahl der zweiten Drahtelemente, d.h. der Drahtelemente mit dem zweiten Winkel (großer Flechtwinkel), höchstens 1:1, insbesondere höchstens 1:2, insbesondere höchstens 1:4, insbesondere höchstens 1:6, insbesondere höchstens 1:8, insbesondere höchstens 1:12, insbesondere höchstens 1:24, betragen. Aufgrund der kleineren Anzahl der ersten Drahtelemente bzw. generell der Drahtelemente mit dem relativ kleineren Winkel werden die ersten Drahtelemente, d.h. jedes einzelne erste Drahtelement, mit einer höheren Kraft gestreckt als die zweiten Drahtelemente bzw. die Drahtelemente mit dem relativ größeren Flechtwinkel gestaucht bzw. komprimiert werden. Die ersten Drahtelemente nehmen dabei axiale Zugkräfte auf und erzeugen dadurch eine über die Gitterstruktur auf eine Gefäßwand wirkende Rückstellkraft. Dieser Effekt wird durch die unterschiedlichen Eigenschaften der Drahtelemente verstärkt. Dadurch, dass die Anzahl der ersten Drahtelemente, die einen kleineren Winkel aufweisen, gegenüber der Anzahl der zweiten Drahtelemente kleiner ist, kann die Rückstellkraft der Gitterstruktur fein eingestellt werden.

Bei der Komprimierung der Gitterstruktur wirken aufgrund des Kräftegleichgewichts auf die zweiten Drahtelemente Druckkräfte, die zu einer axialen Verkürzung bzw. Stauchung der zweiten Drahtelemente führen. Da wenigstens ein zweites Drahtelement, insbesondere mehrere zweite Drahtelemente, einem ersten Drahtelement zugeordnet ist, wird die Belastung der zweiten Drahtelemente begrenzt. Insbesondere wird durch mehrere zweite Drahtelemente pro erstem Drahtelement erreicht, dass die auf die zweiten Drahtelemente wirkenden Druckkräfte, die zum Ausgleich der auf die ersten Drahtelemente wirkenden Zugkräfte bei der Komprimierung entstehen, verteilt werden, so dass auf ein einzelnes zweites Drahtelement eine kleinere Kraftkomponente wirkt.

Eine Dehnung der Drahtelemente ist bei kleinen Winkeldifferenzen zwischen den ersten und zweiten Drahtelementen möglich. Im Unterschied zu symmetrischen Flechtkonfigurationen weisen beispielsweise rohrförmige Gitterstrukturen, insbesondere Stents, durch die asymmetrische Anordnung der Drahtelemente eine höhere Radialkraft auf, da die Drahtelemente mit kleinerem Winkel Drahtspiralen bilden, die bei der Komprimierung des Gittergeflechts gestreckt werden. Die radial wirkende Kompressionskraft wird durch die zweiten Drahtelemente mit dem größeren Flechtwinkel in eine axiale Kraftkomponente überführt, die eine Streckung der ersten Drahtelemente bewirkt. Die durch die Streckung in den ersten Drahtelementen gespeicherte elastische Energie führt bei der Positionierung der medizinischen Vorrichtung in einem Körpergefäß zu einer Erhöhung der Rückstellkraft.

Durch die unterschiedlichen elastischen Eigenschaften der Drahtelemente wird ferner erreicht, dass die Komprimierbarkeit verbessert wird.. Die Gitterstruktur bzw. der Stent kann auf einen sehr kleinen Querschnittsdurchmesser gebracht, d.h. gecrimpt, werden. Dabei wird eine elastische Rückstellkraft durch die Streckung des ersten Drahtelements mit der höheren Dehnbarkeit bereitgestellt, die im implantierten Zustand eine erhöhte Radialkraft bewirkt. Wenn der Unterschied zwischen dem ersten und dem zweiten Winkel α', α" klein ist, insbesondere im Bereich von 2° bis 10°, ist die verhältnismäßige bzw. prozentuale Streckung des ersten Drahtelements relativ klein. Bei großen Winkelunterschieden wird das Drahtelement weiter gestreckt, d.h. der Grad der Verlängerung des ersten Drahtelements ist erhöht. Durch geeignete Wahl der Kombination aus Winkeldifferenz und elastischen Eigenschaften der Drahtelemente ist es möglich, die Radialkraft und die Kompressionskraft bzw. Komprimierbarkeit der Vorrichtung an verschiedene medizinische Anwendungen anzupassen.

Es ist möglich, dass wenigstens ein Stabilisierungsabschnitt vorgesehen ist, der sich zumindest bereichsweise in axialer Richtung entlang der Gitterstruktur erstreckt, wobei der erste Winkel entlang des Stabilisierungsabschnitts stets größer oder stets kleiner als der zweite Winkel ist. Auf diese Weise kann erreicht werden, dass bereichsweise die Radialkraft der Gitterstruktur erhöht ist, während die außerhalb des Stabilisierungsabschnitts angeordneten Abschnitte eine geringere Radialkraft aufweisen.

Der Stabilisierungsabschnitt kann einen axial mittleren Abschnitt und/oder axialen Endabschnitt der Gitterstruktur bilden. Durch die Bildung eines axial mittleren Abschnitts als Stabilisierungsabschnitt kann die Vorrichtung beispielsweise als Implantat, insbesondere Stent, in ein Gefäß eingesetzt werden, wobei der Stabilisierungsabschnitt durch die erhöhte Radialkraft eine (Wieder-)Aufweitung des Gefäßes gewährleistet. Die axialen Endabschnitte weisen in diesem Fall eine geringere Radialkraft auf. Dadurch passen sich die Endabschnitte an die Bewegung der Gefäßwand, die beispielsweise bei Blutgefäßen durch den Pulsschlag bewirkt wird, an.

Ferner können medizinische Vorrichtungen einen ersten axialen Endabschnitt aufweisen, der ein asymmetrisches Geflecht aufweist, und einen zweiten axialen Endabschnitt, der eine symmetrische bzw. flexible Flechtkonfiguration umfasst. Es ist möglich, dass die Dehnbarkeitseigenschaften der Drahtelemente durch geeignete Maßnahmen im zeitlichen Verlauf veränderbar sind, so dass die Gitterstruktur beispielsweise nach der Implantation kurzzeitig eine hohe Radialkraft zur Aufweitung des Gefäßes aufweist und die Radialkraft später reduziert wird, um die mechanische Belastung der geheilten Gefäßwand, d.h. nach erfolgreicher Therapie, zu verringern. Beispielsweise kann das erste Drahtelement ein bioresorbierbares Material aufweisen.

Vorzugsweise variiert der erste Winkel und/oder der zweite Winkel zumindest abschnittsweise entlang der Gitterstruktur, insbesondere entlang einer parallel zur Rotationsachse R verlaufenden Geraden R', R". Die Gitterstruktur umfasst somit unterschiedliche Bereiche mit jeweils unterschiedlicher Winkeländerung zwischen dem ersten und dem zweiten Drahtelement. Die Radialkraft entlang der Gitterstruktur kann auf diese Weise kontinuierlich variieren. Es ist auch möglich, dass die unterschiedlichen Geflechtbereiche derart abgegrenzt sind, dass die Radialkraft entlang der Gitterstruktur stufenweise variiert. Grundsätzlich weisen Bereiche mit einer hohen Radialkraft eine Stabilisierungsfunktion auf und eignen sich insbesondere zur Behandlung von harten Plaques, Stenosen oder zur Fixierung von Coils in Aneurysmen. Bereiche mit einer kleinen Radialkraft bieten eine ausreichend hohe radiale Flexibilität, um sich an die Bewegung der Gefäßwand, beispielsweise in Folge des Pulsschlags, anzupassen. Eine kleinere Radialkraft ist ferner bei der Behandlung von weichen bzw. vulnerablen Plaques (Softplaques) vorteilhaft.

Die rotationssymmetrische Gitterstruktur kann im Wesentlichen rohrförmig, insbesondere stentartig, ausgebildet sein, so dass die Vorrichtung als Implantat zur Stützung von Körpergefäßen, insbesondere Blutgefäßen, einsetzbar ist.

Vorzugsweise sind die Drahtelemente entlang der Gitterstruktur zumindest abschnittsweise coilartig gewunden. Durch die coilartige bzw. spiralförmige Anordnung der Drahtelemente ist eine einfache Herstellung der medizinischen Vorrichtung möglich.

Die Drahtelemente können entlang der Gitterstruktur zumindest abschnittsweise miteinander verflochten sein. Bei einer Flechtstruktur ist jeweils ein Drahtelement wechselweise über und unter wenigstens einem weiteren Drahtelement geführt.

Ferner können die Drahtelemente zumindest an einem axialen Ende oder in einem Kreuzungsbereich der Gitterstruktur miteinander verbunden, insbesondere form-, kraft- und/oder stoffschlüssig, insbesondere verflochten, verdrillt, verklebt oder verschweißt, sein. Beispielsweise können die Drahtelemente an einem axialen Ende der Gitterstruktur miteinander verbunden sein derart, dass die Gitterstruktur im Wesentlichen aus einem zusammenhängenden Draht gebildet ist, der an den axialen Enden jeweils umgelenkt und in die Gegenrichtung geführt ist. Auf diese Weise werden offene Drahtenden an den axialen Enden der Gitterstruktur vermieden und beispielsweise beim Einsatz der Vorrichtung als Stent die Verletzungsgefahr reduziert. Ferner ermöglicht eine Verbindung der Drahtelemente untereinander, insbesondere in einem Kreuzungsbereich, eine weitere, verbesserte Radialkraft der Gitterstruktur. Vorzugsweise sind die Drahtelemente an den axialen Enden des Stabilisierungsabschnitts bzw. der Gitterstruktur verbunden, so dass eine axiale Streckung bzw. elastische Verlängerung des ersten Drahtelements in Abhängigkeit zur Komprimierung der durch das zweite Drahtelement gebildeten Drahtspirale erfolgt.

Vorteilhafterweise begrenzt der Kreuzungsbereich, in dem die Drahtelemente miteinander verbunden sind, den Stabilisierungsabschnitt. Die Kreuzungsbereiche, in denen die Drahtelemente miteinander verbunden sind, ermöglichen eine herstellungsbedingt einfach festlegbare Abgrenzung zwischen dem Stabilisierungsabschnitt und weiteren Abschnitten der Gitterstruktur.

Bei einer bevorzugten Ausführungsform der erfindungsgemäßen, medizinischen Vorrichtung umfassen die Drahtelemente, insbesondere die zweiten Drahtelemente, ein Formgedächtnismaterial, insbesondere eine Nickel-Titan-Legierung. Derartige Materialien weisen eine hohe Beständigkeit und Biokompatibilität auf und ermöglichen außerdem, dass im Rahmen der unterschiedlichen elastischen Eigenschaften eine festlegbare Durchmesseränderung bzw. radial wirkende Kraftkomponente der Gitterstruktur einstellbar ist. Besonders vorteilhaft ist die Verwendung von Formgedächtnismaterialien, beispielsweise Nitinol, oder Polymeren, beispielsweise Polyester, Polyamid, Polyethylen oder Polypropylen, für das erste Drahtelement, das dehnbarer ist als das zweite Drahtelement und einen kleineren Flechtwinkel aufweist. Derartige Materialien ermöglichen einen großen Dehnungsbereich bzw. eine hohe axiale Streckung des ersten Drahtelements, so dass die rotationssymmetrische Gitterstruktur zur Positionierung in einem Zufuhrsystem ausreichend komprimierbar ist.

Die Erfindung wird im Folgenden anhand von Ausführungsbeispielen unter Bezug auf die beigefügten schematischen Zeichnungen näher erläutert. Darin zeigen
- Fig. 1: die Anordnung zweier Drahtelemente des Implantats gemäß einem bevorzugten Ausführungsbeispiel;
- Fig. 2a, 2b: eine Flechtkonfiguration eines erfindungsgemässen Implantats gemäß einem weiteren Ausführungsbeispiel;
- Fig. 3a, 3b,: 3c eine weitere Flechtkonfiguration des erfindungsgemässen Implantats nach einem weiteren Ausführungsbeispiel;
- Fig. 4a, 4b: eine perspektivische Ansicht eines erfindungsgemässen Implantats nach einem weiteren, bevorzugten Ausführungsbeispiel;
- Fig. 5: eine Detailansicht einer Flechtkonfiguration für ein erfindungsgemässes Implantat gemäß einem weiteren Ausführungsbeispiel; und
- Fig. 6: eine Tabelle bevorzugter Materialien des dehnbareren Drahtelements des erfindungsgemässen Implantats.
Die Figuren zeigen in schematischer Weise Ausführungsbeispiele für Implantate, die zur Behandlung von Aneurysmen oder Stenose geeignet sind.

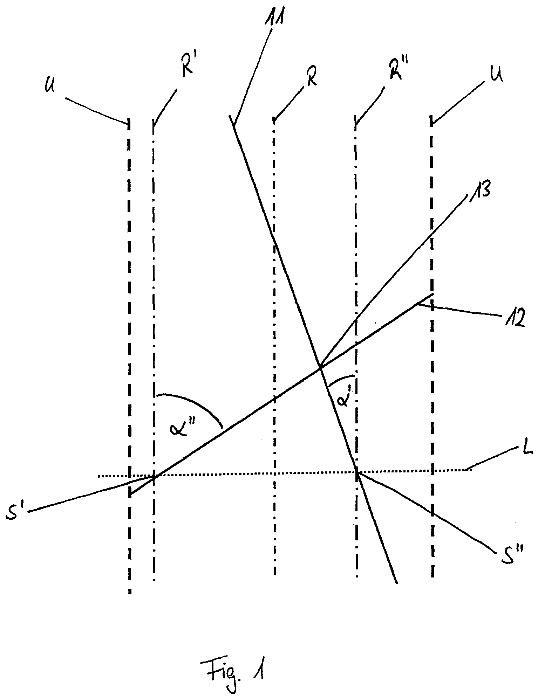

In Fig. 1 ist beispielhaft die Anordnung zweier Drahtelemente 11, 12 eines solchen Implantats gezeigt, wobei insbesondere die Geraden R', R", die Ebene L sowie die durch die Geraden R', R" und die Ebene L gebildeten Schnittpunkte S', S" dargestellt sind. Aus Gründen der Übersichtlichkeit sind in Fig. 1 nur zwei Drahtelemente 11, 12 dargestellt. Grundsätzlich kann die Gitterstruktur 10 mehrere Drahtelemente 11, 12 umfassen. Die Gitterstruktur 10 ist in der Darstellung gemäß Fig. 1 im aufgeklappten Zustand gezeigt, d.h. der Abstand zwischen den Begrenzungslinien U entspricht dem Umfang der Gitterstruktur 10. Die Drahtelemente 11, 12 überlappen sich und bilden dabei einen Kreuzungsbereich 13. Die gemäß Fig. 1 rohrförmige Gitterstruktur ist bezüglich einer Rotationsachse R rotationssymmetrisch ausgebildet. Eine senkrecht zur Rotationsachse R angeordnete Querschnittsebene L bildet mit den Drahtelementen 11, 12 jeweils einen Schnittpunkt S', S". Durch die Schnittpunkte S', S" verläuft jeweils eine Gerade R', R", die parallel zur Rotationsachse R und auf der Umfangsfläche der Gitterstruktur 10 angeordnet ist. Der zwischen dem ersten Drahtelement 11 und der ersten Geraden R' im Bereich des ersten Schnittpunktes S' gebildete Winkel α' entspricht dabei dem Flechtwinkel des ersten Drahtelements 11. Dasselbe gilt für den zweiten Winkel α", der im Bereich des zweiten Schnittpunkts S" durch den Winkel zwischen dem zweiten Drahtelement 12 und der zweiten Geraden R" gebildet ist. Zum Vergleich der Winkel α', α" der beiden Drahtelemente 11, 12 werden grundsätzlich die spitzen Winkel herangezogen, d.h. Winkel mit einem Wert, der wenigstens 0° und weniger als 90° beträgt. Es könnte auch jeweils der stumpfe Winkel verglichen werden.

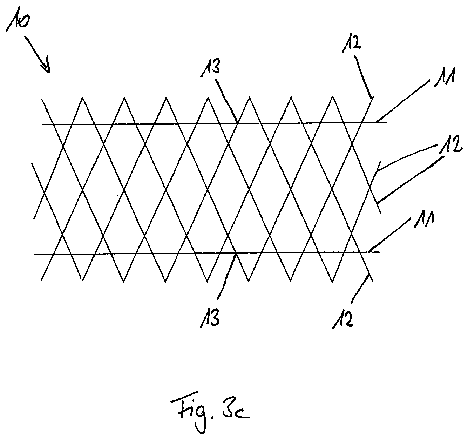

Bei einem Winkel α', α", der 0° beträgt, ist das entsprechende Drahtelement 11, 12 mit der jeweiligen Geraden R', R" fluchtend bzw. parallel zur Rotationsachse R angeordnet und bildet ein in axialer Richtung der Gitterstruktur 10 verlaufendes Band mit unterschiedlichen Eigenschaften (Fig. 3c).

Das erste Drahtelement 11, d.h. das Drahtelement 11, 12 mit dem kleineren Flechtwinkel bzw. Winkel α' ist dehnbarer als das zweite Drahtelement 12. Das kann durch unterschiedliche Werkstoffeigenschaften und/oder unterschiedliche Querschnittsdurchmesser und/oder unterschiedliche Querschnittsprofile erreicht werden. Es handelt sich also um drahtinhärente Eigenschaften.

Im Rahmen der Erfindung wird eine höhere Dehnbarkeit des ersten Drahtelements 11 mit dem kleineren Flechtwinkel α' bevorzugt, wobei nicht ausgeschlossen ist, dass das zweite Drahtelement 12 elastischer ist als das erste Drahtelement 11.

Die Drahtelemente 11, 12 sind im Allgemeinen spiralförmig um die Rotationsachse R gewunden, wobei die Drahtelemente 11, 12 in gleicher Richtung oder gegenläufig entlang der Rotationsachse R angeordnet sein können. Das einzelne Drahtelement 11, 12 weist demnach im Wesentlichen eine spiralfederförmige Struktur auf, wobei die Steigung des spiralförmig gewundenen Drahtelements 11 gegenüber dem Drahtelement 12 aufgrund der unterschiedlichen Winkel α', α" verschieden ist. Die Drahtelemente 11, 12 selbst können einen runden, ovalen oder eckigen Querschnitt aufweisen. Andere Profilquerschnitte sind möglich. Das erste Drahtelement 11 kann einen anderen Querschnitt bzw. ein anderes Querschnittsprofil, insbesondere eine kleinere Querschnittsfläche, aufweisen als das zweite Drahtelement 12. Um eine axiale Dehnung, d.h. Verlängerung, des ersten Drahtelements 11 zu erreichen, sind die Drahtelemente 11, 12 in den axialen Endabschnitten der Gitterstruktur 10 vorzugsweise verbunden. Die Drahtelemente 11, 12 können an ihren Enden, insbesondere den freien Drahtenden 11', 12', oder im Bereich von Kreuzungsbereichen 13 beispielsweise formschlüssig verbunden sein. Die Drahtelemente 11, 12 können auch miteinander verschweißt oder verklebt sein. Es ist auch möglich, dass die in den Kreuzungsbereichen 13 zwischen den Drahtelementen 11, 12 wirkenden Reibungskräfte in Verbindung mit der asymmetrischen Geflechtkonfiguration allein eine feste Verbindung der Drahtelemente 11, 12 bewirken, so dass ein Gleiten der Drahtelemente 11, 12 aufeinander vermieden wird. Zumindest tragen die Reibungskräfte zur Fixierung der Drahtelemente 11, 12 in den Kreuzungsbereichen 13 bei und ermöglichen die Streckung des ersten Drahtelements 11 mit dem kleineren Winkel α'.

Die hier beschriebenen Drahtelemente 11, 12 bilden die Gitterstruktur 10, d.h. die Drahtelemente 11, 12 sind derart spiralförmig auf den Umfang der Gitterstruktur angeordnet, dass der Querschnittsdurchmesser der gebildeten Spirale dem Querschnittsdurchmesser der Gitterstruktur 10 entspricht. Das Lumen der medizinischen Vorrichtung wird demnach durch den Querschnittsdurchmesser der einzelnen Drahtspiralen festgelegt.

Die Erfindung ist nicht auf streng rotationssymmetrische Gebilde eingeschränkt. Vielmehr umfasst die Erfindung Implantate, deren Wandung ein Innenlumen bildet bzw. einen Innenraum, wobei die Wandung auf die mit dieser im Gebrauch in Kontakt kommende Gefäßwand eine nach außen gerichtete Radialkraft ausübt. Dies ist beispielsweise auch bei einem Körper möglich, der einen hohlovalen oder anderen Querschnitt aufweist. Ferner genügt es, wenn das Implantat bzw. die medizinische Vorrichtung zumindest abschnittsweise rotationssymmetrisch bzw. annähernd rotationssymmetrisch ausgebildet ist, wobei weitere Abschnitte des Implantats nicht rotationssymmetrisch ausgebildet sein können.

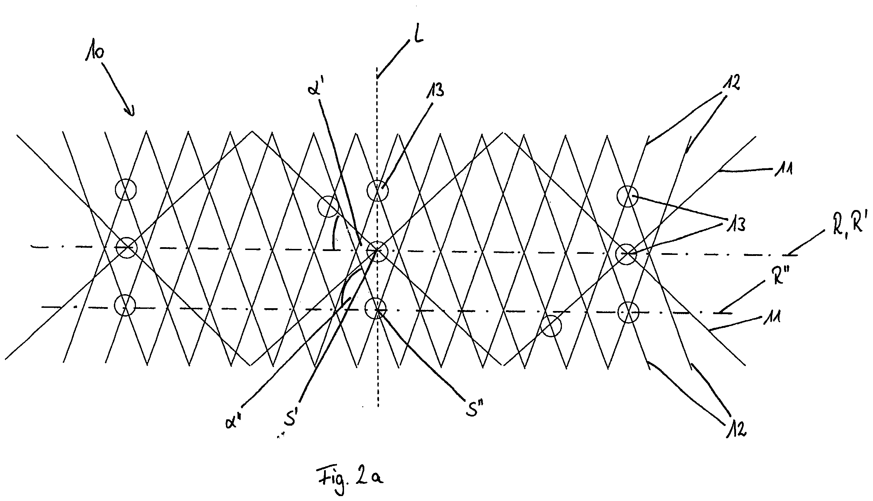

Fig. 2a zeigt ein weiteres Ausführungsbeispiel des erfindungsgemässen Implantats, das mehrere die Gitterstruktur 10 bildende Drahtelemente 11, 12 umfasst. Die Gitterstruktur 10 ist dabei ebenfalls im aufgeklappten Zustand dargestellt, so dass die gesamte Umfangsfläche der Gitterstruktur 10 in der Zeichnungsebene abgebildet ist. Die Gitterstruktur 10 weist zwei erste Drahtelemente 11 auf, die insgesamt vier zweite Drahtelemente 12 überlagern bzw. mit diesen verflochten sind. Eine andere Anzahl von ersten und zweiten Drahtelementen 11, 12 ist möglich. Die ersten Drahtelemente 11 weisen dabei einen kleineren Flechtwinkel α' auf als die zweiten Drahtelemente 12, wobei die Rotationsachse R der Gitterstruktur 10 gemäß Fig. 2a horizontal in der Zeichnungsebene angeordnet ist.

In Fig. 2a sind ferner mehrere Kreuzungsbereiche 13 durch kreisförmige Markierungen hervorgehoben, in denen sich ein erstes Drahtelement 11 mit einem zweiten Drahtelement 12 überschneidet. Es können sich auch mehrere Drahtelemente 11, 12 in einem Kreuzungsbereich 13 überschneiden. Ferner ist eine Ebene L dargestellt, die durch drei Kreuzungsbereiche 13 verläuft, in denen sich jeweils zwei erste Drahtelemente 11 oder zwei zweite Drahtelemente 12 kreuzen. Die Schnittpunkte S', S" zwischen der Ebene L und den Drahtelementen 11, 12 sind in diesem Fall in den Kreuzungsbereichen 13 angeordnet. Der Schnittpunkt S' zwischen den beiden ersten Drahtelementen 11 und der Ebene L liegt dabei auf der Geraden R', die in der Darstellung gemäß Fig. 2a identisch mit der Rotationsachse R ist bzw. die Rotationsachse R überlagert. Die zweite Gerade R" ist parallel zur Rotationsachse R angeordnet und verläuft durch den zweiten Schnittpunkt S", der durch die Ebene L und wenigstens ein, hier zwei, zweite Drahtelemente 12 gebildet ist.

In dem Fall, dass die Ebene L durch einen Kreuzungsbereich 13 eines ersten Drahtelements 11 und eines zweiten Drahtelements 12 verläuft, fallen der erste Schnittpunkt S' und der zweite Schnittpunkt S" sowie die beiden Geraden R', R" zusammen bzw. sind identisch. Die zwischen den Geraden R', R" und den jeweils zugeordneten Drahtelementen 11, 12 gebildeten Winkel α', α" weisen unterschiedliche Werte auf. Insbesondere sind die zweiten Drahtelemente 12 unter einem größeren Flechtwinkel α" angeordnet als die ersten Drahtelemente 11, d.h. die durch die ersten Drahtelemente 11 gebildeten Drahtspiralen weisen eine größere Steigung auf als die durch die zweiten Drahtelemente 12 gebildeten Drahtspiralen. Grundsätzlich sind die Drahtelemente 11, 12 auf demselben Umfang der Gitterstruktur 10 angeordnet, d.h. die durch die Drahtelemente 11, 12 gebildeten spiralförmigen Rotationskörper weisen im Wesentlichen denselben Querschnittsdurchmesser auf. Dabei.können die Drahtelemente 11, 12 miteinander verflochten bzw. verwoben sein oder sich überlagern. Beispielsweise ist ein erstes Drahtelemente 11 auf dem Außenumfang eines zweiten, spiralförmig gewundenen Drahtelements 12 angeordnet, wobei das erste Drahtelement 11 dieselbe Rotationsachse R aufweist wie das zweite Drahtelement 12.

Die ersten Drahtelemente 11 weisen andere elastische Eigenschaften auf als die zweiten Drahtelemente 12. Insbesondere sind die ersten Drahtelemente 11 dehnbarer bzw. elastischer als die zweiten Drahtelemente 12 ausgebildet. Der erhöhte Dehnungsbereich der ersten Drahtelemente 11 kann durch die Werkstoffeigenschaften, beispielsweise den Elastizitätsmodul, erreicht werden. Die für die Dehnung bzw. Streckung der Drahtelemente 11, 12 erforderliche Kraft kann durch die Geometrie, beispielsweise das Querschnittsprofil oder den Querschnittsdurchmesser, der Drahtelemente 11, 12 beeinflusst werden. Beispielsweise ist für die Streckung der ersten Drahtelemente 11 mit einem kleineren Querschnittsdurchmesser eine kleinere Kraft notwendig als für die Dehnung der zweiten Drahtelemente 12 mit einem größeren Querschnittsdurchmesser. Die genannten Möglichkeiten zur Beeinflussung der Eigenschaften der Drahtelemente 11, 12 können miteinander kombiniert werden. Durch die beschriebenen Maßnahmen, einzeln oder in Kombination, wird erreicht, dass die ersten Drahtelemente 11 zur Komprimierung der Gitterstruktur 10 streckbar sind.

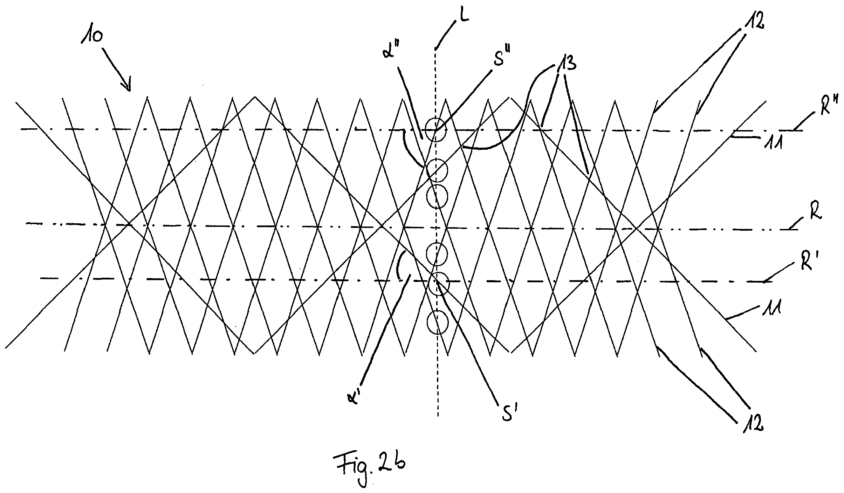

Wie in den Figuren 2a und 2b gut zu erkennen, können die durch die zweiten Drahtelemente 12 gebildeten Drahtspiralen aufgrund des größeren Flechtwinkels α" bei Komprimierung der Gitterstruktur in einem größeren Maß verlängert werden, da die Länge der zweiten Drahtelemente 12 größer ist als die Länge der ersten Drahtelemente 11, die einen kleineren Flechtwinkel α' aufweisen. Dadurch, dass die ersten Drahtelemente 11 dehnbarere bzw. elastischere Eigenschaften, beispielsweise ein elastischeres Material, aufweisen, wirken sie einer Längenänderung der Drahtspiralen aus den zweiten Drahtelementen 12 entgegen. Die zweiten Drahtspiralen 12 werden also durch die elastischen Kräfte der ersten Drahtelemente 11 in den expandierten Zustand zurückgezwungen. Durch diesen Mechanismus wird, in Kombination mit der asymmetrischen Flechtkonfiguration, die gegenüber den bekannten Implantaten bzw. Vorrichtungen erhöhte Rückstell- bzw. Radialkraft erreicht.

Die beiden ersten Drahtelemente 11 sind im Wesentlichen in unterschiedlichen Richtungen um die gemeinsame Rotationsachse R gewunden, so dass sich die beiden ersten Drahtelemente 11, 12 in regelmäßigen Abständen entlang der Gitterstruktur 10 kreuzen. Dasselbe gilt im Wesentlichen für die in den Figuren 2a und 2b gezeigten vier zweiten Drahtelemente 12, wobei jeweils zwei zweite Drahtelemente 12 in derselben Richtung um die gemeinsame Rotationsachse R gewunden sind und jeweils ein Drahtspiralenpaar bilden, das gegenüber dem anderen Drahtspiralenpaar der beiden anderen zweiten Drahtelemente 12 gegenläufig angeordnet ist. Die ersten Drahtelemente 11 und die zweiten Drahtelemente 12 bilden demnach jeweils eine symmetrische Flechtkonfiguration, wobei die Flechtwinkel der jeweiligen symmetrischen Geflechte unterschiedlich sind. Die asymmetrische Anordnung wird durch die Kombination bzw. Überlagerung bzw. das Zusammenflechten der beiden symmetrischen Geflechte erreicht. Durch die jeweils gegenläufige Anordnung der ersten Drahtelemente 11 und der zweiten Drahtelemente 12 wird die Torsionsstabilität des asymmetrischen Gittergeflechts erhöht.

Eine andere Anzahl von ersten und/oder zweiten Drahtelementen 11, 12 ist möglich. Die ersten Drahtelemente 11 mit kleinem Flechtwinkel können 6, 8, 12, 16, 24 Drahtelemente umfassen. Die zweiten Drahtelemente mit großem Flechtwinkel können 8, 12, 24, 32 , 36, 40, 44, 80, 84, 88, 94 Drahtelemente umfassen. Die Drahtelemente 11, 12 können auch alle in derselben Richtung oder in gegenläufiger Richtung gewunden sein oder eine beliebige Kombination der verschiedenen Wicklungsrichtungen umfassen.

Aus den Fig. 2a und 2b ist ersichtlich, dass die asymmetrische Flechtkonfiguration durch die Überlagerung wenigstens zweier in sich symmetrischer Unterstrukturen bzw. Geflechte erfolgt. Bei dem Ausführungsbeispiel gemäß Fig. 2a umfasst die erste Struktur 15 zwei erste Drahtelemente 11, die denselben Flechtwinkel α' aufweisen und zueinander symmetrisch angeordnet sind. Die erste Unterstruktur 15 ist daher in sich symmetrisch aufgebaut. Die zweite Unterstruktur 16 umfasst vier Drahtelemente 12, die jeweils denselben zweiten Flechtwinkel α" aufweisen. Die zweite Unterstruktur 16 ist daher ebenfalls in sich symmetrisch ausgebildet. Die jeweiligen Flechtwinkel α', α" der ersten Unterstruktur 15 und der zweiten Unterstruktur 16 sind voneinander verschieden. Insbesondere ist der erste Flechtwinkel α' der ersten Unterstruktur 15 kleiner als der zweite Flechtwinkel α" der zweiten Unterstruktur 16. Durch die Überlagerung bzw. Überlappung der beiden Unterstrukturen 15, 16 ergibt sich die asymmetrische Gesamtstruktur der Vorrichtung, die konkret daraus hervorgeht, dass die ersten und zweiten Drahtelemente 11, 12 jeweils unterschiedliche Flechtwinkel α', α" aufweisen.

Dasselbe gilt für die Vorrichtung gemäß Fig. 2b, die sich von der Vorrichtung gemäß Fig. 2a im Wesentlichen nur durch die Lage der imaginären Ebene L sowie der imaginären Geraden R', R" unterscheidet.

Es ist auch möglich, dass wenigstens zwei verschiedene Unterstrukturen mit unterschiedlichen Flechtwinkeln miteinander kombiniert sind bzw. zusammenwirken. Die Erfindung ist nicht auf die Kombination zweier in sich symmetrischer Unterstrukturen beschränkt, sondern umfasst die Kombination von mehr als zwei Unterstrukturen, beispielsweise von drei, vier oder mehr Unterstrukturen. Die einzelnen Unterstrukturen sind jeweils in sich symmetrisch mit demselben Flechtwinkel aufgebaut. Die Flechtwinkel der einzelnen Strukturen sind verschieden, so dass beispielsweise ein erster, zweiter, dritter oder vierter Flechtwinkel vorliegt. Die Flechtwinkel der weiteren (mehr als zwei) Strukturen können auch gleich groß sein. Verschiedenen Strukturen können in axialer Längsrichtung der Vorrichtung nachgeordnet sein und eine oder mehrere Strukturen überlagern, wobei die Flechtwinkel der sich jeweils überlagernden Strukturen unterschiedlich sind, so dass die Eigenschaften der Vorrichtung bzw. des Stents, beispielsweise im Hinblick auf die Steifigkeit, die Radialkraft, die Compliance usw. variabel sind.

Fig. 2b zeigt im Wesentlichen dieselbe Gitterstruktur 10 wie Fig. 2a, wobei die Betrachtungsebene L gegenüber der Darstellung gemäß Fig. 2a in axialer Richtung versetzt angeordnet ist. Dabei ist die Betrachtungsebene L derart angeordnet, dass die Ebene L keinen Kreuzungsbereich 13 durchläuft. Die Schnittpunkte S', S" werden daher jeweils von nur einem Drahtelement 11, 12 und der Ebene L gebildet. In Fig. 2b sind die Schnittpunkte S', S" durch kreisförmige Markierungen hervorgehoben. Die Winkel α', α" zwischen den Drahtelementen 11, 12 und den parallel zur Rotationsachse R angeordneten Geraden R', R" sind ebenfalls unterschiedlich. Aus den Fig. 2a und 2b ist ersichtlich, dass die unterschiedlichen Winkel α', α" unabhängig von der Lage der Betrachtungsebene L erkennbar sind. Für die Bestimmung der Winkel α', α" ist allerdings die Anordnung der Referenzgeraden R', R" relevant, d.h. die Betrachtung der spitzen Winkel zwischen den Drahtelementen 11, 12 und der jeweiligen Projektion der Rotationsachse R auf die Umfangsebene im Schnittpunkt S', S".

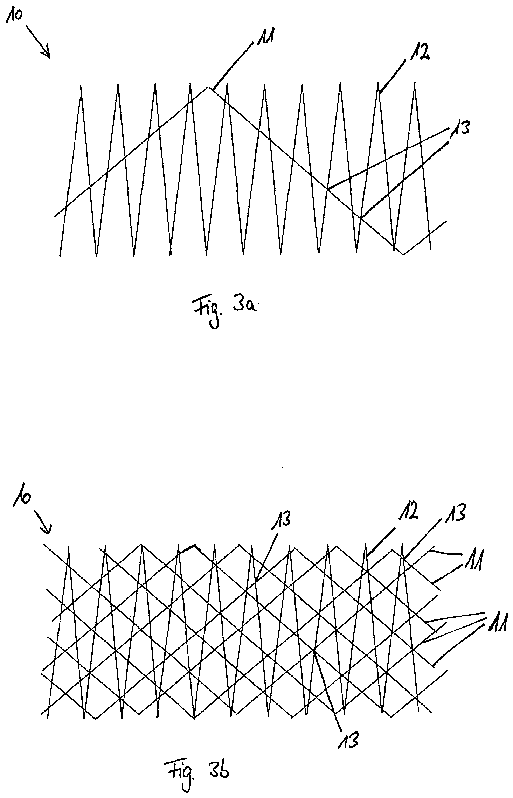

Fig. 3a zeigt ein weiteres Ausführungsbeispiel der erfindungsgemäßen medizinischen Vorrichtung, wobei die Gitterstruktur 10 aus zwei Drahtelementen 11, 12 gebildet ist, die einen unterschiedlichen Winkel α', α" und unterschiedliche elastische Eigenschaften aufweisen. Das erste Drahtelement 11 überlagert dabei das zweite Drahtelement 12. In den Kreuzungsbereichen 13 berühren sich die Drahtelemente 11, 12, wobei das erste Drahtelement 11 sowohl vollständig oberhalb des zweiten Drahtelements 12, als auch vollständig unterhalb des zweiten Drahtelements 12 verlaufen kann. Es ist außerdem möglich, dass das erste Drahtelement 11 das zweite Drahtelement 12 teilweise oberhalb und teilweise unterhalb kreuzen kann, insbesondere derart, dass das erste Drahtelement 11 mit dem zweiten Drahtelement 12 verflochten ist.

Ein weiteres Ausführungsbeispiel zeigt Fig. 3b, wobei das Ausführungsbeispiel im Wesentlichen eine Erweiterung der Gitterstruktur 10 gemäß Fig. 3a darstellt. Dabei überlagert eine Vielzahl von ersten Drahtelementen 11 ein einzelnes zweites Drahtelement 12.

Das zweite Drahtelement 12 kann auch ein oder mehrere erste Drahtelemente 11 überlagern oder mit den ersten und/oder weiteren zweiten Drahtelementen 11, 12 verflochten sein. Die mehreren ersten Drahtelemente 11 können sich ebenfalls überlagern oder miteinander verflochten sein. Unterstützt durch die unterschiedlichen elastischen Eigenschaften können durch die unterschiedliche Anzahl der ersten und zweiten Drahtelemente 11, 12 die Verformungseigenschaften des Gesamtgeflechts beeinflusst werden. Anstelle der unterschiedlichen Anzahl der verschiedenen Drahtelemente kann die Dicke der Drahtelemente erhöht werden, wodurch erreicht wird, dass die Drahtelemente mit der erhöhten Dicke weniger gestaucht werden.

In Fig. 3c ist ein weiteres Ausführungsbeispiel der erfindungsgemäßen Vorrichtung dargestellt, wobei vier zweite Drahtelemente 12 ein im Wesentlichen symmetrisches Gittergeflecht bilden. Dazu sind jeweils zwei zweite Drahtelemente 12 parallel bzw. in gleicher Richtung um eine gemeinsame Rotationsachse gewunden, und zwei weitere zweite Drahtelemente 12 gegenläufig angeordnet. Ferner sind bei der Gitterstruktur 10 gemäß Fig. 3c zwei erste Drahtelemente 11 dargestellt, die im Wesentlichen in axialer Richtung, d.h. unter einem Winkel von 0°, angeordnet sind. Es ist möglich, dass mehrere erste Drahtelemente vorgesehen sind. Die ersten Drahtelemente 11 können mit den zweiten Drahtelementen 12 in den Kreuzungsbereichen 13 verbunden sein. Da die ersten Drahtelemente 11 einen Flechtwinkel bzw. Winkel α' aufweisen, der 0° beträgt, erfolgt bei der Komprimierung der Gitterstruktur 10 gemäß Fig. 3c die Streckung der ersten Drahtelemente 11 im Wesentlichen ohne eine Torsion der ersten Drahtelemente 11. Die ersten Drahtelemente 11 bilden axiale Bänder, die eine besonders gute axiale Stabilität der Gitterstruktur 10 bewirken und durch die unterschiedlichen drahtinhärenten elastischen Eigenschaften (s. Beschreibung zu Fig. 2) eine Einstellung der Stabilität erlauben.

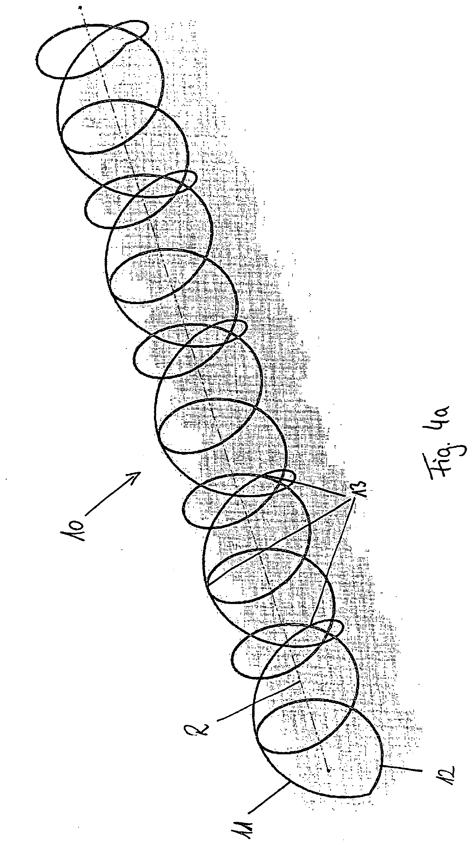

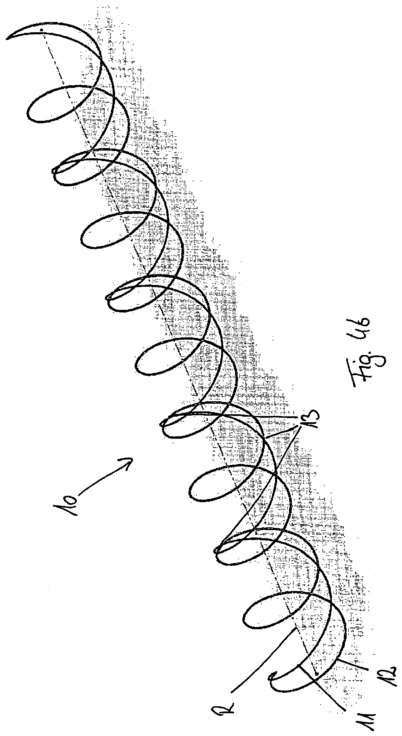

Die Fig. 4a und 4b zeigen ein Ausführungsbeispiel der erfindungsgemäßen medizinischen Vorrichtung mit einer Gitterstruktur 10, die aus zwei Drahtelementen 11, 12 gebildet ist. Dabei weist das erste Drahtelement 11 einen kleineren Winkel α' bezüglich der Rotationsachse R auf als das zweite Drahtelement 12. Das erste Drahtelement 11 ist zudem dehnbarer bzw. elastischer als das zweite Drahtelement 12. In der perspektivischen Darstellung gemäß den Fig. 4a und 4b ist der Winkelunterschied zwischen den Drahtelementen 11, 12 durch die unterschiedliche Steigung der spiralförmigen Drahtelemente 11, 12 erkennbar. Insbesondere ist der Winkelunterschied auch dadurch ersichtlich, dass das erste Drahtelement 11 bei einer Rotation um 360° um die Rotationsachse R eine größere Längserstreckung in axialer Richtung entlang der Gitterstruktur 10 aufweist als eine Rotation bzw. Windung des zweiten Drahtelements 12. An den axialen Enden der Gitterstruktur 10 sind die Drahtelemente 11, 12 miteinander verbunden. Es ist möglich, dass die beiden Drahtelemente 11, 12 aus einem einzigen Draht gebildet sind bzw. an den axialen Enden der Gitterstruktur 10 derart stoffschlüssig verbunden sind, dass die Gitterstruktur 10 im Wesentlichen aus einem einzigen durchgehenden Draht gebildet ist.

Generell können an den axialen Enden der Gitterstruktur 10 zwei oder mehrere Drahtelemente 11, 12 miteinander verbunden sein. Dabei können ein oder mehrere erste Drahtelemente 11 mit einem oder mehreren zweiten Drahtelementen 12 verbunden sein. Es ist auch möglich, dass zwei oder mehrere erste Drahtelemente 11 oder zwei oder mehrere zweite Drahtelemente 12 jeweils miteinander verbunden sind, so dass jeweils nur diejenigen Drahtelemente 11, 12 miteinander verbunden sind, die denselben Flechtwinkel aufweisen. Die Verbindung zwischen den Drahtelementen 11, 12 kann im Allgemeinen kraftschlüssig, formschlüssig oder stoffschlüssig erfolgen. Es ist nicht ausgeschlossen, dass die Drahtelemente 11, 12 mit Hilfe zusätzlicher Verbindungselemente verbunden sind.

Wenn die Drahtelemente 11, 12 unterschiedliche Querschnittsdurchmesser oder -profile aufweisen, kann der Übergang zwischen den Drahtelementen 11, 12 im Bereich der stoffschlüssigen oder andersartigen Verbindung im Wesentlichen fließend bzw. kontinuierlich sein. Bei dem Ausführungsbeispiel gemäß Fig. 4a sind die Drahtelemente 11, 12 am axialen Ende der Gitterstruktur unter einem stumpfen Winkel verbunden, so dass die durch die Drahtelemente 11, 12 gebildeten Drahtspiralen im Wesentlichen gegenläufig angeordnet sind. Hingegen bildet die Verbindung zwischen den Drahtelementen 11, 12 am axialen Ende der Gitterstruktur 10 gemäß Fig. 4b einen spitzen Winkel, d.h. die Drahtelemente 11, 12 sind entlang der Rotationsachse R der Gitterstruktur 10 im Wesentlichen in gleicher Richtung angeordnet, wobei die Drahtspiralen unterschiedliche Steigungen aufweisen. Die Drahtelemente 11, 12 können allgemein an den axialen Enden der Gitterstruktur 10 formschlüssig verbunden sein. Die Drahtelemente 11, 12 können Abschlussknoten bilden. Andere Verbindungsarten sind möglich. Die Drahtelemente 11, 12 können auch in einem mittleren Abschnitt der Gitterstruktur 10 verbunden bzw. zueinander fixiert sein.

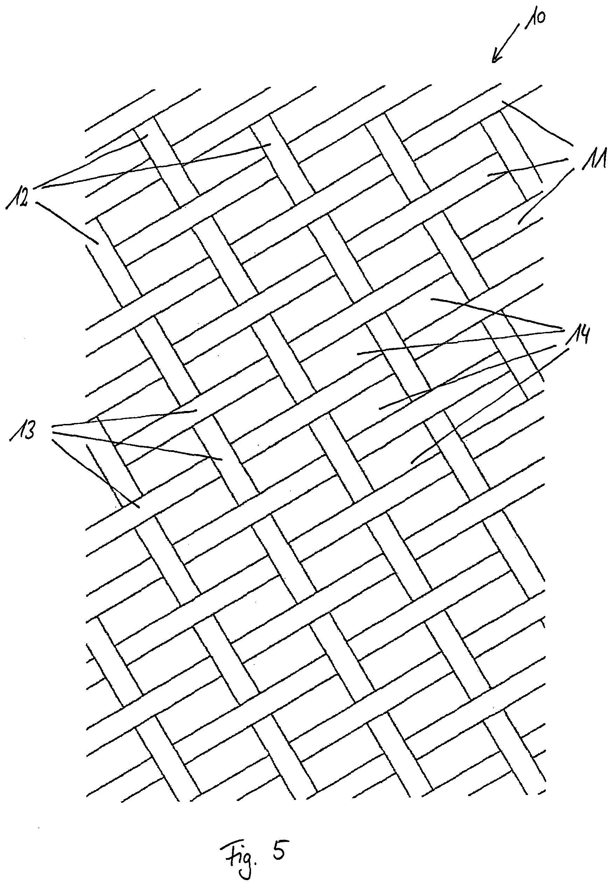

Eine Detailansicht einer asymmetrischen Flechtkonfiguration gemäß einem weiteren Ausführungsbeispiel der erfindungsgemäßen medizinischen Vorrichtung zeigt Fig. 5. Das dargestellte Gittergeflecht bzw. die Gitterstruktur 10 weist mehrere erste Drahtelemente 11 und mehrere zweite Drahtelemente 12 auf, wobei die ersten Drahtelemente 11 in der Zeichnungsebene diagonal von links unten nach rechts oben und die zweiten Drahtelemente 12 diagonal von rechts unten nach links oben verlaufen. Die Rotationsachse R (nicht dargestellt) verläuft in Fig. 5 vertikal in der Zeichnungsebene.

Die Asymmetrie, d.h. die unterschiedlichen Flechtwinkel der Drahtelemente 11, 12 ist dadurch erkennbar, dass die geflochtenen Drahtelemente 11, 12 keine rautenförmigen oder quadratischen Zellen 14 bilden. Vielmehr bilden die Drahtelemente 11, 12 rechteckförmige Zellen 14, d.h. die Drahtelemente 11, 12 bilden in den Kreuzungsbereichen 13 jeweils einen rechten Winkel, wobei die Diagonalen der Zellen 14 nicht senkrecht, sondern schräg zueinander angeordnet sind. Andere geometrische Ausgestaltungen der Zellen 14 sind möglich.

Die Drahtelemente 11, 12 sind ferner miteinander verflochten, wobei jedes erste Drahtelement 11 jeweils einmal unter und einmal über ein zweites Drahtelement 12 geführt ist. Andere Flechtkonfigurationen sind ebenfalls möglich, beispielsweise können zwei oder mehrere erste Drahtelemente 11 über und unter ein oder mehrere Drahtelemente 12 geführt werden.

Die Drahtelemente 11, 12 weisen gemäß Fig. 5 im Wesentlichen dieselbe Geometrie und denselben Querschnittsdurchmesser auf. Die elastischen Eigenschaften der Drahtelemente 11, 12 sind also durch die Werkstoffeigenschaften bestimmt.

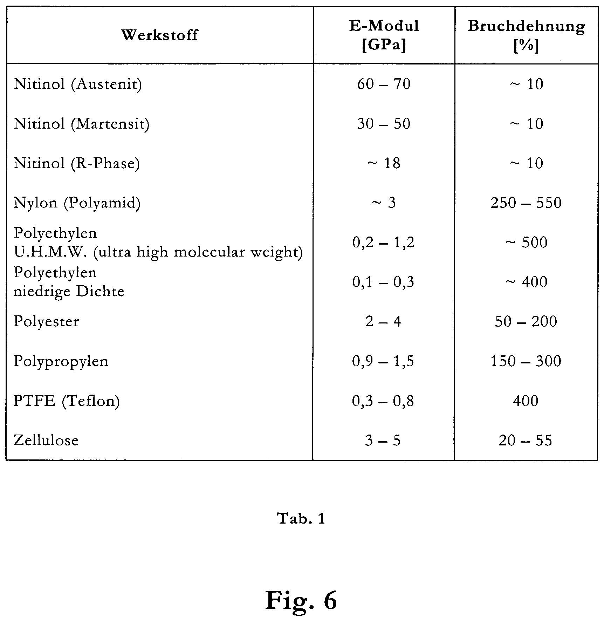

Besonders bevorzugte Werkstoffe zur Erhöhung der Elastizität des ersten Drahtelements 11 gegenüber dem zweiten Drahtelement 12 sind Polymere, wie beispielsweise Polyester, Polyamid, Polyethylen, HDPE, Polypropylen oder PTFE. Weitere mögliche Werkstoffe für das erste, elastische Drahtelement mit bevorzugten Wertebereichen des Elastizitätsmoduls (E-Modul) und der Bruchdehnung des jeweiligen Werkstoffs sind in Tab. 1 (Fig. 6) angegeben, wobei die Erfindung nicht auf die genannten Materialien eingeschränkt ist. Kombinationen verschiedener Materialien sind möglich.

Die zweiten Drahtelemente 12 weisen vorzugsweise ein Formgedächtnismaterial, beispielsweise eine Nickel-Titan-Legierung wie Nitinol auf. Es ist möglich, dass das erste Drahtelement 11 und das zweite Drahtelement 12 eine Nickel-Titan-Legierung oder eine der genannten Polymerverbindungen umfasst. Der Einsatz einer Nickel-Titan-Legierung für das erste Drahtelement 11 hat den Vorteil, dass die Zugkraft zur Streckung des ersten Drahtelements 11 ab einem gewissen Dehnungsgrad im Wesentlichen konstant bleibt. Insbesondere weisen Nickel-Titan-Legierungen bis zu einem bestimmten Dehnungsgrad ein E-Modul auf, der größer ist als der E-Modul, der bei größerer Dehnung bzw. Streckung besteht. Durch die asymmetrische Flechtkonfiguration erreicht das erste Drahtelement 11 mit dem kleineren Winkel α' durch die relativ große Längenänderung dieses Plateau der Kraft-Dehnungs-Eigenschaften. Da die zweiten Drahtelemente 12 aufgrund des größeren Flechtwinkels bzw. Winkels α" nicht oder nur geringfügig gestreckt werden, ist der E-Modul der zweiten Drahtelemente 12 größer. Die Dehnung der ersten Drahtelemente 11 im Bereich des Kräfteplateaus hat den Vorteil, dass die Kraft relativ gleichmäßig und konstant, auch bei unterschiedlichen Gefäßdurchmessern oder bei Änderung des Gefäßdurchmessers, beispielsweise durch Pulsschlag, auf die Gefäßwand wirkt.

Es ist möglich, dass die medizinische Vorrichtung, insbesondere das Gittergeflecht 10, teilweise ein asymmetrisches Geflecht aufweist. Beispielsweise kann die Gitterstruktur 10 Stabilisierungsabschnitte umfassen, die in den Randbereich der Gitterstruktur 10 angeordnet sind. Der Mittelbereich der Gitterstruktur 10 kann ein symmetrisches Geflecht umfassen.

Bei einer Gitterstruktur 10, die einen Stabilisierungsabschnitt und zumindest einen weiteren Abschnitt aufweist, kann ein Übergangsbereich zwischen dem Stabilisierungsabschnitt und dem weiteren Abschnitt einen kontinuierlichen Übergang bilden. Die Drahtelemente 11, 12 können im Übergangsbereich zueinander lose bzw. nur reibschlüssig verbunden sein. Um die Wirkung der elastischen Eigenschaften des ersten Drahtelementes 11 auf den Stabilisierungsabschnitt zu begrenzen, ist es bevorzugt, die Drahtelemente 11, 12 im Übergangsbereich zu verbinden. Die asymmetrische Konfiguration der Gitterstruktur 10 wird dabei unverändert beibehalten.

Es ist möglich, dass sich der Flechtwinkel bzw. Winkel α', α" der Drahtelemente 11, 12 in axialer Richtung entlang der Gitterstruktur 10 bzw. der Rotationsachse R ändert. Sowohl der erste Winkel α', der dem ersten Drahtelement 11 zugeordnet ist, als auch der zweite Winkel α", der dem zweiten Drahtelement 12 zugeordnet ist, oder beide Winkel α', α" können variiert werden. Auf diese Weise können unterschiedliche Radialkraftbereiche entlang der Gitterstruktur 10 eingestellt werden. Die Gitterstruktur 10 kann also unterschiedliche Bereiche aufweisen, die sich durch ihre Rückstell- bzw. Radialkraft voneinander unterscheiden. Die Übergänge zwischen den einzelnen Bereichen können kontinuierlich bzw. fließend, insbesondere stufenlos, sein. Die einzelnen Bereiche können auch scharf voneinander abgegrenzt sein. Dies kann beispielsweise dadurch erreicht werden, dass die Drahtelemente 11, 12 in einer, vorzugsweise derselben, Querschnittsebene eine Biegung aufweisen bzw. gebogen sind, so dass der Flechtwinkel α', α" in einem ersten Bereich einen anderen Wert aufweist als in einem zweiten, benachbarten Bereich der Gitterstruktur 10. Eine derartige Umlenkung bzw. Biegung der Drahtelemente 11, 12 erfolgt vorzugsweise in einem Kreuzungsbereich 13, wobei die Drahtelemente 11, 12 in diesen Kreuzungsbereich 13 miteinander verbunden sein können.

Vorteilhafterweise ändert sich der kleinere Winkel α' entlang der Gitterstruktur 10. Dadurch kann die Radialkraft entlang des Stents bzw. der Gitterstruktur 10 besonders einfach variiert bzw. variabel eingestellt werden. Eine derartige Variation des ersten Winkels α' kann beispielsweise derart gestaltet sein, dass der erste Winkel α', der dem dehnbareren bzw. elastischen ersten Drahtelement 11 zugeordnet ist, im Mittelbereich, d.h. in einem axial mittleren Bereich, der Gitterstruktur 10 kleiner ist als in einem Randbereich. Dadurch wird im Mittelbereich der Gitterstruktur 10 eine höhere Radialkraft bereitgestellt, die beispielsweise beim Einsatz der medizinischen Vorrichtung als Stent eine zuverlässige Aufweitung und Stabilisierung des Blutgefäßes ermöglicht. In den Randbereichen der Gitterstruktur 10 wird durch einen relativ größeren Winkel α' des ersten Drahtelements 11 eine verbesserte Anpassung der Gitterstruktur 10 an die sich durch den Pulsschlag bewegenden Gefäßwand ermöglicht (Compliance).

Durch eine entsprechende Änderung des Flechtwinkels in den Randbereichen ist es auch möglich, in den axialen Randbereichen eine hohe Radialkraft einzustellen, wodurch das Implantat im Gefäß gut fixiert wird. Zwischen den Randbereichen können, auch abschnittsweise, Bereiche vorgesehen sein, bei denen durch eine entsprechende Einstellung des Flechtwinkels eine vergleichsweise geringere Radialkraft auf die Gefäßwand ausgeübt wird, um zu vermeiden, dass, beispielsweise bei Softplaque keine Beschädigungen auftreten.

Es ist ferner möglich, dass die Gitterstruktur 10 eine Umhüllung, d.h. ein Graft, aufweist. Die Umhüllung erstreckt sich zumindest abschnittsweise in Umfangsrichtung und/oder axialer Richtung entlang der Gitterstruktur. Die Umhüllung kann sowohl auf einem äußeren, als auch auf einem inneren Umfang der Gitterstruktur angeordnet sein oder die Drahtelemente vollständig umhüllen derart, dass die Gitterstruktur zumindest abschnittsweise vollständig in die Umhüllung eingebettet ist. Die Umhüllung ist vorzugsweise mit der Gitterstruktur verbunden und zumindest bereichsweise flexibel. Die Umhüllung umfasst vorzugsweise einen Kunststoff, beispielsweise Polyurethan, Silikon oder Teflon.

### Bezugszeichenliste

- 10: Gitterstruktur
- 11: erstes Drahtelement
- 12: zweites Drahtelement
- 13: Kreuzungsbereich
- 14: Zelle
- 15,16: Unterstrukturen

- R: Rotationsachse
- R': erste Gerade
- R": zweite Gerade
- S': erster Schnittpunkt
- S": zweiter Schnittpunkt
- α': erster Winkel
- α": zweiter Winkel

## Patentansprüche

1. Implantat aufweisend eine komprimierbare rotationssymmetrische Gitterstruktur (10) mit wenigstens zwei spiralförmig um eine gemeinsame Rotationsachse R gewundenen Drahtelementen (11, 12), die mit einer gemeinsamen, senkrecht zur Rotationsachse angeordneten Ebene L jeweils einen ersten und zweiten Schnittpunkt S', S" bilden, wobei durch den ersten Schnittpunkt S' eine erste Gerade R' und durch den zweiten Schnittpunkt S" eine zweite Gerade R" verlaufen, die jeweils parallel zur Rotationsachse R angeordnet sind und mit jeweils einem der beiden Drahtelemente (11, 12) einen spitzen Winkel (α', α") einschließen,
**dadurch gekennzeichnet, dass**
die beiden Winkel (α', α") unterschiedlich sind und die beiden Drahtelemente (11, 12) unterschiedliche elastische Eigenschaften aufweisen.

2. Implantat nach Anspruch 1,
**dadurch gekennzeichnet, dass**
die beiden Drahtelemente (11, 12) wenigstens ein erstes Drahtelement (11) und wenigstens ein zweites Drahtelement (12) umfassen, wobei das erste Drahtelement (11) mit der ersten Geraden R' einen kleineren Winkel α' einschließt als das zweite Drahtelement (12) mit der zweiten Geraden R".

3. Implantat nach Anspruch 2,
**dadurch gekennzeichnet, dass**
das erste Drahtelement (11) dehnbarer ist als das zweite Drahtelement (12).

4. Implantat nach Anspruch 2 oder 3,
**dadurch gekennzeichnet, dass**
das erste Drahtelement (11) ein kleineres Verhältnis zwischen Zugkraft und Dehnung aufweist als das zweite Drahtelement (12).

5. Implantat nach wenigstens einem der Ansprüche 1 bis 4,
**dadurch gekennzeichnet, dass**
das erste Drahtelement (11) einen kleineren Elastizitätsmodul aufweist als das zweite Drahtelement (12).

6. Implantat nach Anspruch 5,
**dadurch gekennzeichnet, dass**
das Verhältnis zwischen dem Elastizitätsmodul des ersten Drahtelements (11) und dem Elastizitätsmodul des zweiten Drahtelements (12) höchstens 1:10, insbesondere höchstens 1:100, insbesondere höchstens 1:1000, beträgt.

7. Implantat nach Anspruch 5 oder 6,
**dadurch gekennzeichnet, dass**
der Elastizitätsmodul des ersten Drahtelements (11) zwischen 100 MPa und 5000 MPa, insbesondere zwischen 200 MPa und 4000 MPa, insbesondere zwischen 300 MPa und 3000 MPa, insbesondere zwischen 500 MPa und 2000 MPa, insbesondere zwischen 800 MPa und 1500 MPa, insbesondere zwischen 900 MPa und 1200 MPa, beträgt.

8. Implantat nach Anspruch 2 oder 3,
**dadurch gekennzeichnet, dass**
das erste Drahtelement (11) eine Bruchdehnung von mehr als 10 %, 30 %, 50 %, 100 %, 150 %, 200 % aufweist.

9. Implantat nach wenigstens einem der Ansprüche 1 bis 8,
**dadurch gekennzeichnet, dass**
das erste Drahtelement (11) eine kleinere Querschnittsfläche, insbesondere einen kleineren Querschnittsdurchmesser, aufweist als das zweite Drahtelement (12).

10. Implantat nach wenigstens einem der Ansprüche 1 bis 9,
**dadurch gekennzeichnet, dass**
die beiden Drahtelemente (11, 12) unterschiedliche Querschnittsprofile aufweisen.

11. Implantat nach Anspruch 10,
**dadurch gekennzeichnet, dass**
das erste Drahtelement (11) ein flaches, insbesondere rechteckiges oder ovales, Querschnittsprofil aufweist derart, dass das erste Drahtelement (11) im Wesentlichen bandförmig ausgebildet ist.

12. Implantat nach wenigstens einem der Ansprüche 1 bis 11,
**dadurch gekennzeichnet, dass**
der Winkelunterschied zwischen dem ersten Winkel (α') und dem zweiten Winkel (α") wenigstens 2°, insbesondere wenigstens 5°, insbesondere wenigstens 8°, insbesondere wenigstens 10°, insbesondere wenigstens 20°, insbesondere wenigstens 30°, insbesondere wenigstens 40°, insbesondere wenigstens 45°, insbesondere wenigstens 50°, insbesondere wenigstens 60°, insbesondere wenigstens 70°, beträgt.

13. Implantat nach wenigstens einem der Ansprüche 1 bis 12,
**dadurch gekennzeichnet, dass**
der Winkelunterschied zwischen dem ersten Winkel (α') und dem zweiten Winkel (α") höchstens 10°, insbesondere höchstens 8°, insbesondere höchstens 6°, insbesondere höchstens 4°, insbesondere höchstens 2°, beträgt.

14. Implantat nach wenigstens einem der Ansprüche 1 bis 13,
**dadurch gekennzeichnet, dass**
das Verhältnis zwischen der Anzahl der ersten Drahtelemente (11) und der Anzahl der zweiten Drahtelemente (12) höchstens 1:1, insbesondere höchstens 1:2, insbesondere höchstens 1:4, insbesondere höchstens 1:6, insbesondere höchstens 1:8, insbesondere höchstens 1:12, insbesondere höchstens 1:24, beträgt.

## Claims

1. An implant having a compressible rotationally symmetrical mesh structure (10) with at least two wire elements (11, 12) coiled in a spiral around a common axis of rotation R, each wire element forming a first and second point of intersection S', S" together with a common plane L arranged perpendicularly to the axis of rotation, wherein a first straight line R' passes through the first point of intersection S' and a second straight line R" passes through the second point of intersection S", each line being parallel to the axis of rotation R and forming an acute angle (α', α") to each of the two wire elements (11, 12),
**characterized in that**
the two angles (α', α") are different, and the two wire elements (11, 12) have different elastic properties.

2. The implant according to claim 1,
**characterized in that**
the two wire elements (11, 12) comprise at least one first wire element (11) and at least one second wire element (12), wherein the first wire element (11) forms a smaller angle a' with the first straight line R' than the second wire element (12) with the second straight line R".

3. The implant according to claim 2,
**characterized in that**
the first wire element (11) is more expandable than the second wire element (12).

4. The implant according to claim 2 or 3,
**characterized in that**
the first wire element(11) has a smaller ratio between the tensile force and the elongation than the second wire element (12).

5. The implant according to at least one of claims 1 to 4,
**characterized in that**
the first wire element (11) has a lower modulus of elasticity than the second wire element (12).

6. The implant according to claim 5,
**characterized in that**
the ratio between the modulus of elasticity of the first wire element (11) and the modulus of elasticity of the second wire element (12) amount to at most 1:10, in particular at most 1:100, in particular at most 1:1000.

7. The implant according to claim 5 or 6,
**characterized in that**
the modulus of elasticity of the first wire element (11) is between 100 MPa and 5000 MPa, in particular between 200 MPa and 4000 MPa, in particular between 300 MPa and 3000 MPa, in particular between 500 MPa and 2000 MPa, in particular between 800 MPa and 1500 MPa, in particular between 900 MPa and 1200 MPa.

8. The implant according to claim 2 or 3,
**characterized in that**
the first wire element (11) has an elongation at fracture of more than 10 %, 30 %, 50 %, 100 %, 150 %, 200 %.

9. The implant according to at least one of claims 1 to 8,
**characterized in that**
the first wire element (11) has a smaller cross-sectional area, in particular a smaller cross-sectional diameter than the second wire element (12).

10. The implant according to at least one of claims 1 to 9,
**characterized in that**
the two wire elements (11, 12) have different cross-sectional profiles.

11. The implant according to claim 10,
**characterized in that**
the first wire element (11) has a flat cross-sectional profile, in particular a rectangular or oval cross-sectional profile such that the first wire element (11) is designed essentially in the form of a strip.

12. The implant according to at least one of claims 1 to 11,
**characterized in that**
the angular difference between the first angle (α') and the second angle (α") amounts to at least 2°, in particular at least 5°, in particular at least 8°, in particular at least 10°, in particular at least 20°, in particular at least 30°, in particular at least 40°, in particular at least 45°, in particular at least 50°, in particular at least 60°, in particular at least 70°.

13. The implant according to at least one of claims 1 to 12,
**characterized in that**
the angular difference between the first angle (α') and the second angle (α") amounts to at most 10°, in particular at most 8°, in particular at most 6°, in particular at most 4°, in particular at most 2°.

14. The implant according to at least one of claims 1 to 13,
**characterized in that**
the ratio between the number of the first wire elements (11) and the number of the second wire elements (12) amounts to at most 1:1, in particular at most 1:2, in particular at most 1:4, in particular at most 1:6, in particular at most 1:8, in particular at most 1:12, in particular at most 1:24.

## Revendications

1. Implant présentant une structure grillagée compressible symétrique en rotation (10) comportant au moins deux éléments en fil (11, 12) enroulés en spirale autour d'un axe de rotation commun R, qui constituent respectivement avec un plan L disposé perpendiculairement à l'axe de rotation un premier et un second point de coupe S', S", sachant que, à travers le premier point de coupe S', passe une première droite R' et à travers le second point de coupe S" une seconde droite R" qui sont disposées respectivement parallèlement à l'axe de rotation R et circonscrivent un angle aigu (α', α") avec un des deux éléments en fil (11, 12),
**caractérisé en ce que**
les deux angles (α', α") sont différents et les deux éléments en fil (11, 12) présentent des propriétés élastiques différentes.

2. Implant selon la revendication 1,
**caractérisé en ce que**
les deux éléments en fil (11, 12) comprennent au moins un premier élément en fil (11) et au moins un second élément en fil (12), le premier élément en fil (11) circonscrivant avec la première droite R' un angle plus petit α' que le second élément en fil (12) avec la seconde droite R".

3. Implant selon la revendication 2,
**caractérisé en ce que**
le premier élément en fil (11) est plus extensible que le second élément en fil (12).

4. Implant selon la revendication 2 ou 3,
**caractérisé en ce que**
le premier élément en fil (11) présente un rapport plus faible entre la force de traction et l'extension que le second élément en fil (12).

5. Implant selon au moins une des revendications 1 à 4, **caractérisé en ce que**
le premier élément en fil (11) présente un module d'élasticité plus faible que le second élément en fil (12).

6. Implant selon la revendication 5,
**caractérisé en ce que**
le rapport entre le module d'élasticité du premier élément en fil (11) et le module d'élasticité du second élément en fil (12) est au plus de 1:10, en particulier au plus de 1:100, en particulier au plus de 1:1000.

7. Implant selon la revendication 5 ou 6,
**caractérisé en ce que**
le module d'élasticité du premier élément en fil (11) se situe entre 100 MPa et 5000 MPa, en particulier entre 200 MPa et 4000 MPa, en particulier entre 300 MPa et 3000 MPa, en particulier entre 500 MPa et 2000 MPa, en particulier entre 800 MPa et 1500 MPa, en particulier entre 900 MPa et 1200 MPa.

8. Implant selon la revendication 2 ou 3,
**caractérisé en ce que**
le premier élément en fil (11) présence une extension à la rupture supérieure à 10 %, 30 %, 50 %, 100 %, 150 %, 200 %.

9. Implant selon au moins une des revendications 1 à 8,
**caractérisé en ce que**
le premier élément en fil (11) présente une surface de section transversale inférieure, en particulier un diamètre de section transversale inférieur, à ceux du deuxième élément en fil (12).

10. Implant selon au moins une des revendications 1 à 9,
**caractérisé en ce que**
les deux éléments en fil (11, 12) présentent des profils de section transversale différents.

11. Implant selon la revendication 10,
**caractérisé en ce que**
le premier élément en fil (11) présente un profil de section transversale plat, en particulier rectangulaire ou ovale, de sorte que le premier élément en fil (11) a sensiblement une conformation en bande.

12. Implant selon au moins une des revendications 1 à 11,
**caractérisé en ce que**
la différence d'angle entre le premier angle (α') et le second angle (α") s'élève à au moins 2°, en particulier au moins 5°, en particulier au moins 8°, en particulier au moins 10°, en particulier au moins 20°, en particulier au moins 30°, en particulier au moins 40°, en particulier au moins 45°, en particulier au moins 50°, en particulier au moins 60°, en particulier au moins 70°.

13. Implant selon au moins une des revendications 1 à 12,
**caractérisé en ce que**
la différence d'angle entre le premier angle (α') et le second angle (α") s'élève à au plus 10°, en particulier au plus 8°, en particulier au plus 6°, en particulier au plus 4°, en particulier au plus 2°.

14. Implant selon au moins une des revendications 1 à 13,
**caractérisé en ce que**
le rapport entre le nombre de premiers éléments en fil (11) et le nombre de seconds éléments en fil (12) s'élève à au plus 1:1, en particulier au plus 1:2, en particulier au plus 1:4, en particulier au plus 1:6, en particulier au plus 1:8, en particulier au plus 1:12, en particulier au plus 1:24.
